# EUROPEAN PATENT SPECIFICATION

(11) **EP 3 784 149 B1**
(45) Date of publication and mention of the grant of the patent: **25.11.2026**
(21) Application number: 19724257.1
(22) Date of filing: 24.04.2019
(51) Int. Cl.: A61B 17/12, A61B 17/064

(54) **SUTURING CLIP**
NAHTKLAMMER
CLIP DE SUTURE

(30) Priority: 25.04.2018 US 201862662266 P
(43) Date of publication of application: 03.03.2021
(73) Proprietor: Endomatic Ltd., 6037604 Or Yehuda (IL)
(72) Inventor: KEREN, Dvir, 6233302 Tel Aviv (IL); SCHWARZ, Boaz, 6920503 Tel-Aviv (IL); MARKOVITZ, Shimrit, 6274315 Tel Aviv (IL)
(74) Representative: RGTH
(86) International application number: PCT/IL2019/050461
(87) International publication number: WO 2019/207585

(56) References cited:
- EP-A1- 2 656 816
- EP-A2- 1 512 383
- WO-A1-03/101310
- US-A1- 2007 156 123
- US-A1- 2007 198 057

## Description

### RELATED APPLICATIONS

This application claims the benefit of priority under 35 USC § 119(e) of U.S. Provisional Patent Application No. 62/662,266 filed April 25, 2019.

### FIELD AND BACKGROUND OF THE INVENTION

The present invention, in some embodiments thereof, relates to the field of closure devices for medical use, and more particularly, to closure devices for use in the LAA.

The medical community considers the left atrium appendage (LAA) as a potential causal locus for CVA (cardiovascular accident) due to the potential of the LAA for embolic creation.

Closing off the LAA may be performed, for example in an open thoracic approach or by a minimally invasive trans-vascular (and typically trans-septal) approach. In the open approach, a surgeon is likely to suture the ostium (the connection between the LAA and the left atrium). In a trans-vascular approach, an interventional cardiologist doesn't open the patient's chest, and seals the LAA, *e*.*g*., using a plug and/or stent-like construction deployed inside the LAA. The principle is that reduction of atrial wall irregularities and/or circulatory dead zones may reduce a potential for thrombogenesis.

Additional background documents include International Patent Publication No. WO2003101310A1 disclosing a clip for closing a puncture hole in a blood vessel comprising a ring having a resiliently expandable circumference and a plurality of barbed prongs extending at least approximately in the same direction from one edge of the ring. A device for deploying such a clip is also described.

European Patent application No. EP1512383A2 disclosing a vascular system comprising a balloon catheter and a vascular device. The balloon catheter has an elongated shaft and an expandable balloon. The vascular device is mounted over the expandable balloon and is composed of shape memory material. The vascular device has a collapsed position and a memorized position and is expandable to an expanded position to engage the vessel walls and returnable substantially to the memorized position to bring the walls radially inwardly.

U.S. Patent application No. US2007/156123A1 disclosing a robotically controlled guide instrument system, comprising an elongate flexible guide instrument; a drive assembly coupled to a proximal portion of the guide instrument and configured to maneuver a distal portion of the guide instrument; a master controller including a user interface that may be manipulated to actuate the drive assembly and thereby maneuver the distal portion the guide instrument; and a closure device detachably coupled to a delivery member carried by the guide instrument, the guide instrument maneuverable to position the closure device proximate an opening of a left atrial appendage of a person's heart such that the prosthesis may be detached from the delivery member and implanted to substantially close off the left atrial appendage.

European Patent application No. EP2656816A1 disclosing a vascular device having a plurality of struts having a distal portion and a proximal portion. The distal portions of the struts are retained in a converged position. The struts diverge radially outwardly. A plurality of vessel penetrating members extend from the proximal portion of the struts for engaging the internal wall of a vessel, wherein release of the retention of the distal portions of the struts causes the distal portions to move outwardly away from the longitudinal axis and the proximal portions of the struts to move inwardly toward the longitudinal axis such that the vessel engaging members pull the internal wall of the vessel radially inwardly.

U.S. Patent application No. US2007/198057A1 disclosing a device for closing holes tissue that is delivered via a catheter to the inside of a body lumen such as a heart. An elastic barbed clip is expanded, pulled into the tissue and released, pulling the tissue with it. The operation is fully reversible.

### SUMMARY OF THE INVENTION

The present invention is defined by the features of the independent claim. Embodiments of the invention are defined in the dependent claims.

There is provided, in accordance with some embodiments of the present disclosure, a suturing clip for closing a body lumen aperture, comprising: a plurality of arms each joined at a basal side of the arm to a core of the suturing clip; and a respective anchor at a terminal end of each arm; wherein the suturing clip is configured to convert between: a collapsed delivery configuration; an expanded anchoring configuration having a diameter at least 5x larger than a diameter of the suturing clip in the collapsed delivery configuration, and a collapsed suturing configuration shaped to re-position the anchors where they close tissue of an aperture perimeter after the tissue is attached by contact with the anchors in the expanded anchoring configuration; wherein the suturing clip, in the collapsed delivery configuration, is configured to attach to a delivery mount and be advanced on the delivery mount into the body lumen aperture by distal movement along a proximal-to-distal axis of the suturing clip; and wherein the anchors of the suturing clip, in the collapsed suturing configuration, are positioned proximal to the core along the proximal-to-distal axis.

In some embodiments, the suturing clip resiliently self-expands from the collapsed delivery configuration to the expanded anchoring configuration.

In some embodiments, the delivery mount is configured to be detached from the suturing clip in the collapsed suturing configuration and withdrawn through tissue closed by the anchors of the suturing clip to the side of the closed tissue opposite the suturing clip.

In some embodiments, the suturing clip is configured to resiliently self-collapse from the expanded anchoring configuration to the collapsed suturing configuration.

In some embodiments, the suturing clip is configured to resiliently self-collapse from the expanded anchoring configuration to the collapsed suturing configuration.

In some embodiments, the suturing clip in the collapsed delivery configuration is sheathed within the sheathing lumen.

In some embodiments, the suturing clip is configured to resiliently self-expand from the collapsed delivery configuration to the expanded anchoring configuration upon a partial unsheathing from the sheathing lumen.

In some embodiments, the suturing clip is configured to resiliently self-collapse from the expanded anchoring configuration to the collapsed suturing configuration upon a further unsheathing from the sheathing lumen.

In some embodiments, the suturing clip is configured to evert during converting between the collapsed delivery configuration and the expanded anchoring configuration, so that a side of the anchors which face radially inward in the collapsed delivery configuration faces outward in the expanded anchoring configuration.

In some embodiments, the suturing clip is configured to evert during converting between the collapsed delivery configuration and the collapsed suturing configuration, so that a portion of the arms which is initially on a distal side of the core moves to a proximal side of the core.

In some embodiments, the suturing clip collapses from the expanded anchoring configuration to the collapsed suturing configuration upon a movement of the closure disk.

In some embodiments, the closure disk moves from a position nearer to the core of the suturing clip to a position further from the core to collapse the suturing clip from the expanded anchoring configuration to the collapsed suturing configuration upon a movement of the closure disk.

In some embodiments, conversion from the expanded anchoring configuration to the collapsed suturing configuration comprises a reverting movement of the arms in a direction opposite a direction of their expansion from the collapsed delivery configuration to the expanded anchoring configuration.

In some embodiments, the suturing clip is provided together with a spreader, separately actuatable to extend laterally from the trans-vascular catheter, insert to the body lumen aperture, and is configured to contact tissue of the body lumen aperture with grips to set a position of the sheathing lumen from which the suturing clip is deployed.

In some embodiments, the suturing clip is unsheathed by a proximal movement of the sheath relative to the suturing clip, and the suturing clip is oriented in the sheath along a proximal-distal axis, with the core on a proximal side of the suturing clip, and the anchors on a distal side of the suturing clip.

In some embodiments, the suturing clip is unsheathed by a proximal movement of the sheath relative to the suturing clip, and the suturing clip is oriented in the sheath along a proximal-distal axis, with the anchors on a proximal side of the suturing clip, and the core on a distal side of the suturing clip.

In some embodiments, the anchors, in the collapsed suturing configuration, define a zigzag pattern extending along a band of closure of the closed tissue.

In some embodiments, the anchors, in the collapsed suturing configuration, draw tissue to a surgical purse-string configuration.

In some embodiments, the arms extend from the core, each from an individual base attached to the core.

In some embodiments, a first plurality of the arms extends from the core via a trunk which is shared in common by the plurality of the arms.

In some embodiments, arms of the first plurality are configured to interdigitate with arms of the second plurality in the collapsed suturing configuration.

In some embodiments, a material of the arms comprises a nitinol alloy.

In some embodiments, a material of the suturing clip comprises a radiopaque marker more radiopaque than nitinol.

In some embodiments, at least one of the arms is mounted to at least one side of the torsion bar, and the torsion bar is configured to twist to move the at least one of the arms from the collapsed delivery configuration toward the collapsed final configuration.

In some embodiments, at least one of the arms is configured to bend along a longitudinal extent of the arm to move the at least one of the arms from the collapsed delivery configuration toward the collapsed final configuration.

There is provided, in accordance with some embodiments of the present disclosure, a suturing clip for closing a body lumen aperture, comprising: a plurality of arms each joined at a basal side of the arm to a core of the suturing clip; a respective anchor at a terminal end of each arm; and a closure disk; wherein the suturing clip is configured to convert between: a collapsed delivery configuration sized to be sheathed by a sheathing lumen of a trans-vascular catheter; an expanded anchoring configuration having a diameter at least 5x larger than a sheathed diameter of the suturing clip in the collapsed delivery configuration, and a collapsed suturing configuration shaped to re-position the anchors where they close tissue of an aperture perimeter after the tissue is attached by contact with the anchors in the expanded anchoring configuration; and wherein the closure disk is movable along a distal-proximal axis of the suturing clip to actuate conversion between expanded anchoring configuration and the collapsed suturing configuration.

There is provided, in accordance with some embodiments of the present disclosure, a suturing clip for closing a body lumen aperture, comprising: a plurality of arms each joined at a basal side of the arm to a core of the suturing clip; and a respective anchor at a terminal end of each arm; and wherein the suturing clip is configured to convert between: an expanded anchoring configuration positioning at least two anchors on one side of a perimeter, and at least one anchor on another side of the perimeter, and a collapsed suturing configuration repositioning the anchors from the expanded anchoring configuration by movement of the at least one anchor to cross between the at least two anchors.

In some embodiments, the at least two anchors comprises at least four anchors, and the at least one anchors comprises at least three anchors; and the collapsed suturing configuration repositions the anchors from the expanded anchoring configuration by moving each of the at least three anchors to cross between respective pairs of the at least four anchors.

There is provided, in accordance with some embodiments of the present disclosure, a suturing clip for closing a body lumen aperture, comprising: a plurality of arms each joined at a basal side of the arm to a core of the suturing clip; and a respective anchor at a terminal end of each arm; wherein the suturing clip is configured to convert between: a collapsed delivery configuration expandable to attach the anchors to tissue of the body lumen aperture, and a collapsed suturing configuration shaped to re-position the anchors where they close the body lumen aperture by movement of the attached tissue; and wherein the anchors are distal to the core in the collapsed delivery configuration, and proximal to the core in the collapsed suturing configuration, while the core remains in a same proximal-to-distal orientation in the two configurations.

In some embodiments, the arms are configured to evert while re-positioning the anchors between the collapsed delivery configuration and the collapsed suturing configuration.

In some embodiments, the anchors are positioned on a radially inward side of the arms in the collapsed delivery configuration, and move to a radially outward side of the arms during movement of the arms toward the collapsed suturing configuration.

There is provided, in accordance with some embodiments of the present disclosure, a device for deploying within a body lumen aperture, comprising: a plurality of arms each joined at a basal side of the arm to a core of the device; and a respective terminal end of each arm; wherein the device is configured to convert between: a collapsed delivery configuration with the terminal ends in a first configuration; a collapsed final configuration shaped to re-position the terminal ends to a second configuration; and a torsion bar; wherein at least one of the arms is mounted to at least one side of the torsion bar, and the torsion bar is configured to twist to move the at least one of the arms from the collapsed delivery configuration toward the collapsed final configuration.

In some embodiments, the device is a suturing clip comprising a respective anchor at each terminal end; and wherein: the collapsed delivery configuration is expandable to attach the anchors to tissue of the body lumen aperture; and the collapsed suturing configuration re-positions the anchors where they close the body lumen aperture by movement of the attached tissue.

In some embodiments, the torsion bar is located where the at least one of the arms are joined at the basal side of the arm to the core.

In some embodiments, the torsion bar is located between two segments of the at least one of the arms.

There is provided, in accordance with some embodiments of the present disclosure, a method of closing a body lumen aperture, comprising: advancing a suturing clip attached to a delivery mount through the body lumen aperture from a first side of the body lumen aperture to a second side of the body lumen aperture; expanding the suturing clip at least partially on the second side of the body lumen aperture; anchoring tissue defining a perimeter of the body lumen aperture to anchors of the suturing clip; and collapsing the suturing clip so that the anchors close the body lumen aperture while the suturing clip remains on the second side of the closed body lumen aperture.

In some embodiments, the suturing clip remains entirely on the second side of the closed body lumen aperture.

In some embodiments, the suturing clip is unexposed to contents of the body lumen on the first side of the closed body lumen aperture after the collapsing.

In some embodiments, the method comprises withdrawing the delivery mount through the closed body lumen aperture to the first side of the body lumen aperture, while the suturing clip remains on the second side of the body lumen aperture, and the body lumen aperture remains closed by the collapsed suturing clip.

In some embodiments, the body lumen aperture is an ostium of a left atrial appendage.

In some embodiments, the advancing comprises advancing the delivery mount through a trans-vascular route to reach the first side of the body lumen aperture.

In some embodiments, the advancing comprises advancing the delivery mount through a heart septal wall to reach the first side of the body lumen aperture.

In some embodiments, the first side is an atrial side of an ostium of an LAA, and the second side is within the LAA.

In some embodiments, the collapsing closes the only aperture leading to the second side of the body lumen aperture.

In some embodiments, the expanding comprises unsheathing at least a portion of the arms of the suturing clip and allowing the arms to resiliently self-expand.

In some embodiments, the collapsing comprises unsheathing a portion of the arms of the suturing clip and allowing the arms to resiliently self-collapse.

In some embodiments, the collapsing comprises operating a closure disk to force the arms to a less-expanded diameter.

In some embodiments, the collapsing comprises moving the anchors into a zigzag configuration.

In some embodiments, the collapsing comprises moving the anchors toward a common radial center.

In some embodiments, the collapsing comprises moving at least one of the anchors radially outward, while at least one of the anchors moves radially inward.

Unless otherwise defined, all technical and/or scientific terms used herein have the same meaning as commonly understood by one of ordinary skill in the art to which the present disclosure pertains. Although methods and materials similar or equivalent to those described herein can be used in the practice or testing of embodiments of the present disclosure, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the present disclosure are herein described, by way of example only, with reference to the accompanying drawings. With specific reference now to the drawings in detail, it is stressed that the particulars shown are by way of example, and for purposes of illustrative discussion of embodiments of the present disclosure. In this regard, the description taken with the drawings makes apparent to those skilled in the art how embodiments of the present disclosure may be practiced.
In the drawings:
FIG. 1A schematically illustrates a suturing clip engaged to tissue within a LAA, according to some embodiments of the present disclosure;
FIG. 1B schematically illustrates the suturing clip of *Figure 1A* in an expanded configuration suitable for tissue engagement, according to some embodiments of the present disclosure;
FIG. 1C schematically illustrates a suturing clip which has engaged tissue within an LAA and then undergone a conformational change to close an LAA ostium, according to some embodiments of the present disclosure;
FIG. 1D schematically illustrates the suturing clip of in the LAA-closing conformation of *Figure 1C*, according to some embodiments of the present disclosure;
FIG. 1E schematically illustrates a trans-septal approach insertion of a suturing clip using a delivery system, according to some embodiments of the present disclosure;
FIG. 1F is a schematic flowchart of a method of inserting of a suturing clip using a delivery system, according to some embodiments of the present disclosure;
FIG. 1G is a schematic flowchart of closure of a body lumen aperture by a suturing clip which closes the aperture from a side of the closure opposite a side from which the suturing clip is introduced, according to some embodiments of the present disclosure;
FIGs. 2A-2I schematically illustrate stages in the deployment of a suturing clip, according to some embodiments of the present disclosure;
FIGs. 2J-2L schematically illustrate movements of anchoring positions over the course of a transition of a suturing clip between a deployed- and-anchored configuration, and a collapsed suturing configuration, according to some embodiments of the present disclosure;
FIGs. 2M-2P illustrate details of interactions between a delivery system and a suturing clip, according to some embodiments of the present disclosure;
FIG. 3A schematically illustrates an expanded configuration of a reverting core-and-arm assembly of a suturing clip, according to some embodiments of the present disclosure;
FIG. 3B schematically illustrates a suturing clip an expanded configuration, including closure disk, according to some embodiments of the present disclosure;
FIG. 3C schematically illustrates a suturing clip in a re-collapsed suturing configuration, collapsed by movement of closure disk, according to some embodiments of the present disclosure;
FIG. 3D schematically illustrates a suturing closure disk, according to some embodiments of the present disclosure;
FIG. 3E schematically illustrates an expanded configuration of a reverting core-and-arm assembly of a suturing clip, according to some embodiments of the present disclosure;
FIGs. 3F-3G schematically illustrate a suturing clip an expanded configuration, including closure disk, according to some embodiments of the present disclosure;
FIG. 3H schematically illustrates a suturing clip in a re-collapsed suturing configuration, collapsed by movement of closure disk, according to some embodiments of the present disclosure;
FIG. 3I schematically illustrates a suturing closure disk, according to some embodiments of the present disclosure;
FIG. 4A schematically illustrate an expanded configuration of a core-and-arm assembly of a suturing clip, according to some embodiments of the present disclosure;
FIG. 4B schematically illustrates a suturing clip an expanded configuration, including closure disk, according to some embodiments of the present disclosure;
FIG. 4C schematically illustrates a suturing closure disk, according to some embodiments of the present disclosure;
FIGs. 4D-4H show deployment of a suturing clip under control of a deployment system, including deployment member, according to some embodiments of the present disclosure;
FIGs. 5A-5D schematically illustrate parts of an everting suturing clip, according to some embodiments of the present disclosure;
FIGs. 5E-5F schematically illustrate an assembled suturing clip in an expanded configuration, according to some embodiments of the present disclosure;
FIGs. 5G-5H schematically illustrate an assembled suturing clip in an everted configuration, according to some embodiments of the present disclosure;
FIGs. 6A-6D schematically illustrate torsion bar arm mountings, according to some embodiments of the present disclosure;
FIGs. 7A-7F schematically illustrate a suturing clip in three different stages of deployment, each from two different viewing angles, according to some embodiments of the present disclosure;
FIG. 7G schematically illustrates a portion of a suturing clip (only one arm is shown) comprising an arm attached to a core, in which bending of arm is around one or more torsion bars, including torsion bars which are positioned along the longitudinal extent of the arm, according to some embodiments of the present disclosure;
FIGs. 8A-8G schematically illustrate an everting-type closure suturing clip comprising arm subassemblies, according to some embodiments of the present disclosure;
FIGs. 9A-9F illustrate a sequence of operations wherein a tissue spreader is used together with a delivery system to assist access to LAA for insertion of a suturing clip, according to some embodiments of the present disclosure;
FIGs. 10A-10C schematically illustrate aspects of design of various tissue spreaders, according to some embodiments of the present disclosure; and
FIGs. 11A-11G schematically illustrate an everting-type closure suturing clip comprising arm subassemblies, according to some embodiments of the present disclosure.

### DESCRIPTION OF SPECIFIC EMBODIMENTS OF THE INVENTION

The present invention, in some embodiments thereof, relates to the field of closure devices for medical use, and more particularly, to closure devices for use in the LAA.

### Overview

An aspect of some embodiments of the present disclosure relates to suturing clips configured for closure of an aperture from an opposite side of the aperture than the side from which the suturing clip is introduced to the aperture, and methods of use thereof.

In some embodiments, a suturing clip is configured to close a left atrial appendage (LAA) in a heart. Closure of the LAA is performed in some patients for mitigation of the thrombogenic potential of the LAA. The suturing clips, in some embodiments, operate to close the LAA from a position ultimately situated entirely within the LAA. This provides a potential advantage by mimicking an advantage of the suturing technique of LAA closure; insofar as it closes the ostium without (or with minimal) artificial material left exposed inside the atrium wall. Artificial material is potentially thrombogenic, increasing a risk for a CVA and/or creating an indication for supporting treatment by Coumadin or another anticoagulant.

The clip also provides the potential advantages of placement via a trans-vascular (minimally invasive) approach. Using a catheter, the suturing clip is introduced (e.g., via a trans-septal approach) into the LAA. It is then expanded inside the LAA and attached to the LAA opening (ostium). Then the clip is collapsed to a suturing configuration which closes the LAA. Upon collapse to the suturing configuration, the suturing clip remains inside the LAA, isolated from the general circulation. The LAA is transformed thereby into a closed lumen, with no open passageway leading into or out of a pocket defined by tissue of the LAA< and in which the suturing clip is contained.

In some embodiments, flexible components of suturing clips (including flexible arms and/or hinges of such suturing clips) are constructed from a superelastic alloy such as nitinol.

Superelasticity is associated with resilient deformation up to an unusually high maximum strain (*e.g.,* 7%-13%); considering that superelastic materials nonetheless have a high elastic modulus (resistance to elastic deformation) typical of a structural metal such as titanium or steel; for example, an elastic modulus in the range of 20-100 GPA. This provides superelastic materials with the potential to store considerable energy by their deformation (*e.g*., energy convertible to closing force), particularly compared to constructions of similar dimensions made from other materials.

Without commitment to a particular mechanism or theory, superelasticity in metal alloys is understood to comprise an elastically resilient response to applied stress, related to reversible movements during phase transformation of a crystal; *e*.*g*., between the austenitic and martensitic phases of the crystal.

Herein, the "superelastically set" shape, configuration, and/or position of a component refers to the shape the unconstrained component tends to assume. The shape may be set (sometimes also referred to as "memorized") during manufacture, for example, by bracing the component in its targeted shape, and heating it above the critical temperature for resetting its superelastic shape.

Superelasticity is also referred to as "pseudoelasticity". Superelastic alloys may additionally have a shape-memory property, whereby the superelasticity is evidenced within a certain range of conditions (*e.g*., a temperature range). Outside that range, the alloy is more deformable; but when brought back into the superelastic range, the alloy returns to its superelastically set shape.

The common name "nitinol" refers to a range of superelastic nickel-titanium alloys (instances of which have different ratios of nickel and titanium) with well-accepted properties of biocompatibility. Some superelastic materials may include additional elements, and/or be based on a different system of alloyed elements. For example superelastic alloys based on the systems Fe-Ni-Al, Cu-Zn-Al, Fe-Mn-Si, and Cu-Al-Ni (optionally in combination with further elements) have been described. Superelastic materials incorporating polymers (*e.g*., polymer-coated nitinol) have also been described. Insofar as other examples and/or types of superelastic materials may become available, embodiments of the current disclosure are optionally adapted to use them.

Accordingly, the high-strain resilience of superelastic components allows large conformational changes. Their high elastic modules causes those conformational changes to proceed while exerting resilient force that can be significant enough to drag attached tissue along with, and/or otherwise applied, for example to assist in gaining an anchoring purchase on tissue. Suturing clips, during deployment to a target aperture such as the ostium of an LAA, pass between:
- A collapsed delivery configuration, small enough for trans-vascular delivery, for example, small enough to fit in a delivery tube having a lumen diameter 5 mm in diameter or less, 4 mm in diameter or less, 3 mm in diameter or less, and/or to fit within another diameter, optionally in a range of about 3-5 mm.
- An expanded anchoring configuration, which positions tissue anchoring elements (anchors) where they can be attached to tissue. In some embodiments, the expanded anchoring configuration expands to a maximum of at least 15 mm, 20 mm, 25 mm, 30 mm, or another diameter. In some embodiments, the expanded anchoring configuration is at least 4x, 5x, 6x, 7x, 8x, 9x, 10x, or another factor larger than a diameter of the suturing clip in the collapsed delivery configuration.
- A collapsed suturing configuration, which brings the tissue anchoring elements toward one another to create a closure, for example as a band-type, or a surgical purse-string-type closure.

In some embodiments, elements undergoing conformation changes comprise arms, attached via a common base or "core" of the suturing clip. An attached side of an arm is also referred to herein as a "basal side" of the arm. The arms may themselves be flexible, and/or they may be attached to the core via a flexible element such as a torsion spring. The suturing clip comprises, for example, 2-20 arms; for example, 2, 5, 7, 10, 13, 15 or another number of arms.

In some embodiments, one or more of the transformations is configured so that it is self-actuated by resilient forces acting from within the superelastic material of the suturing clip. For example, the suturing clip is resiliently self-expanding from the collapsed delivery configuration to the expanded anchoring configuration, and/or resiliently self-collapsing from the expanded anchoring configuration to the collapsed suturing configuration. The resilient forces tend to restore positions of the arms (and in particular, the anchors they carry) to a superelastically set shape of the suturing clip, optionally in one or more stages of expansion and/or collapse.

In some embodiments, one or more of the transformations is initiated by the addition, removal, and/or movement of a constraining device. For example, resilient self-expanding and/or self-collapse of the suturing clip is optionally initiated by at least partial unsheathing of the suturing clip from a sheath (acting as the constraining device). In some embodiments, expansion and/or collapse is induced by movement of a controlling component, for example a closure disk which is attached to the arms in a way that restricts their expansion diameter, *e*.*g*., by passage of each arm through an aperture of the closure disk. As the closure disk moves proximally or distally (for example), the arms are constrained to close or open.

In some embodiments, the transformations are actuated by operation of a relatively long and narrow control member, which passes through the aperture targeted for closure and attaches to the suturing clip. The suturing clip, once collapsed to its collapsed suturing configuration, remains on a distal side of the closure, opposite the side from which it was originally introduced. The control member is withdrawn to that opposite side when closure is complete. The small gap forced by the control member's earlier presence is closed over when it withdraws, due to closure forces exerted by the collapsed suturing clip.

While examples of the use of the suturing clips are presented herein with special reference to closure of the LAA, it should be understood that clips are optionally used, changed as necessary, for closure of other body lumens; for example, appendix, fistulas, and/or gastric restriction. Embodiments of suturing clips may also be used, for example, in reverse-side suturing of wounds and/or surgical incisions and/or punctures. For example, a GI lumen may be closed by a suturing clip deployed via intraperitoneal (e.g, laparoscopic) access, optionally with later removal of the suturing clip via an endoluminal (*e.g*., endoscopic) access. Conversely, deployment of a suturing clip is optionally performed via an endoluminal access to an outside of the lumen, and retrieval (if performed) is from an access outside the lumen. This may be useful, for example, as a planned and/or emergency method of using access from the "wrong side" of an opening to nevertheless achieve closure of that opening. In another example, skin may be reverse-side sutured with potential advantages for security of attachment and/or later aesthetic appearance, optionally with later retrieval of the reverse-side suturing clip via laparoscopic access from another access port. Closure may be performed jointly from two sides of an aperture, and optionally one direction of access. In some embodiments, suturing clips are configured for creating closures in non-surgical situations, for example to effect hidden repairs on fabrics and/or membranous articles, and/or position and clamp such articles for receiving such repairs.

In some embodiments, arms of a suturing clip comprise anchors, configured to engage with tissue, so that force exerted on the anchors moves the tissue into new positions. Optionally, the anchors are shaped as barbs, hooks, spikes, or other shapes. In some embodiments, anchors operate by hinging or flexing differently depending on direction of motion (in the style, for example, of a toggling harpoon). For example, an anchor may be hinged or otherwise constructed so that upon receiving force in a direction for insertion, it holds stiff, but upon receiving a tugging force in the opposite direction, the hinge or flex activates, so that a portion of the anchor turns or expands and can no longer be easily dragged out of the hole through which it entered. Additionally or alternatively, an anchor may be hinged and/or flex so that it becomes radially smaller as it advances into a self-created aperture upon insertion, but otherwise expands and/or tends to be forced into a radially expanded state upon the exertion of force in a reverse direction.

A potential advantage of an actuated suturing clip, compared, e.g., to suturing by needle and thread, is decreased complexity for deployment. With relatively few fine control actions necessary, the surgeon's task is potentially made easier, more reliable, and/or performable with a reduced requirement for training and/or experience.

An aspect of some embodiments of the present disclosure relates to suturing clips configured for reverting closure of an aperture from an opposite side of the aperture than the side from which the suturing clip is introduced to the aperture. In some embodiments, reverting closure closes the only aperture leading to the side of the body lumen aperture in which the suturing clip remains, sealing it within a closed chamber.

In some embodiments, arms of a suturing clip inserted to a body lumen are expanded from a first, collapsed configuration to reach a second, expanded configuration. In the expanded configuration, the suturing clip is manipulated to recruit tissue of the body lumen by attachment thereto. Optionally, recruitment is by attachment of anchors positioned by the expanded arms to a predefined pattern, *e.g.,* to a pattern complementary to a shape of the tissue being anchored in, and/or to a pattern which can be manipulated by relatively simple and/or stereotyped movements in order to achieve reliable attachment.

From the expanded configuration, the device is actuated to achieve closure. Closure, in some embodiments, comprises movement of the attachment points to a new configuration, by a motion which reverses (reverts) a direction of arm and/or anchor movement during expansion to the expanded configuration.

In some embodiments, the movement of the attachment points is actuated by manipulation of a constraining device, such as a disk, ring, or other form of clamp, which constrains a maximum radius of radial expansion as it moves along a length of the device.

An aspect of some embodiments of the present disclosure relates to suturing clips configured for everting closure of an aperture from an opposite side of the aperture than the side from which the suturing clip is introduced to the aperture. In some embodiments, everting closure closes the only aperture leading to the side of the body lumen aperture in which the suturing clip remains, sealing it within a closed chamber.

In some embodiments, arms of a suturing clip inserted to a body lumen are expanded from a first, collapsed configuration to reach a second, expanded configuration. In the expanded configuration, the suturing clip is manipulated to recruit tissue of the body lumen by attachment thereto. Optionally, recruitment is by attachment of anchors positioned by the expanded arms to a predefined pattern, *e.g.,* to a pattern complementary to a shape of the tissue being anchored in, and/or to a pattern which can be manipulated by relatively simple and/or stereotyped movements in order to achieve reliable attachment.

In some embodiments, anchors anchor in tissue with a success rate of less than complete anchoring, *e*.*g*., at least 75% anchoring, at least 80% anchoring, at least 85% anchoring, or another rate of anchoring success. In case one or more arms fail to anchor, the remaining anchors are potentially still sufficient to bring tissue of an aperture opening to a suitable closed configuration.

From the expanded configuration, the device is actuated to achieve closure. Closure, in some embodiments, comprises movement of the attachment points to a new configuration, by a motion which continues a direction of arm and/or anchor movement during expansion to the expanded configuration, resulting in an eversion of the initial collapsed delivery configuration. In some embodiments, the eversion comprises an arm portion such as an anchoring portion which is initially (for example) on a distal side of a core (base) of the suturing clip moves to a position on a proximal side of the core.

In some embodiments, additionally or alternatively, the eversion comprises an anchoring side of the arms which faces radially outward in the expanded anchoring state, but faced radially inward in the collapsed delivery state.

An aspect of some embodiments of the present disclosure relates to suturing clips configured for closure of an aperture in tissue to a closed band.

In some embodiments, suturing clips operate by moving a plurality of anchoring positions attached to a perimeter of the aperture from an open-aperture configuration to a closed-aperture configuration.

In some embodiments, movements of the plurality of anchoring positions comprise an interdigitation of anchoring positions moving from one side of the initial perimeter with anchoring positions moving from an opposite side of the initial perimeter. In some embodiments, the interdigitation comprises one or more anchors from one side of the initial perimeter (and/or a supporting member such as an arm that holds the anchors) crossing a geometrical line extending between two anchors from the other side of the initial perimeter.

In some embodiments, the closed band of the perimeter assumes a zigzag shape. For example, the local lateral extremes of the zigzag (the zigzag's angle vertices or "points") along one side of the zigzag correspond to anchors initially attached to a side of the aperture perimeter, and the local lateral extremes of the zigzag along the other side of the zigzag correspond to anchors initially attached to an opposite side of the aperture perimeter. In some embodiments, relative anchor positions relative to a central longitudinal axis of the zigzag are exchanged during closure, so that anchors from one side of the perimeter come to occupy positions on the opposite side of the axis upon closure. In some embodiments a zigzag shape comprises at least two segments extending between three anchoring sites, *e.g.,* a chevron or "V" shape. In some embodiments, a zigzag shape comprises three or more segments (optionally, N segments, with N > **2)** extending between four or more anchoring sites (optionally, N+1 anchoring sites, each anchoring site corresponding to an anchor which is successfully anchored in tissue). In some embodiments, N+1 is a number of anchoring sites between 3 and 20. Optionally, vertices of the zigzag formed near each anchoring site are rounded.

A closed-aperture configuration provides a potential advantage for sealing and/or strengthening of the aperture closure over time, insofar as a relatively small extent of new endothelial tissue needs to be generated across the closed aperture compared, *e.g.,* to a plug-type closure.

In some embodiments, movements of the plurality of anchoring positions comprise movement of at least one anchor radially outward from the initial perimeter, while other anchors move radially inward. By movement of the anchors, the perimeter is thereby both stretched in one direction, and collapsed in another direction, similar to the effect of flexible loop stretched between two oppositely-situated pulling forces.

An aspect of some embodiments of the present disclosure relates to the use of torsion bars (also referred to herein as "torque beams") as springs to provide actuating force to movements of flexible suturing clip elements such as arms and/or anchors mounted to the arms.

In some embodiments, torsion bars are provided by cutting into the material of a core and/or arm (*e.g.,* laser cutting) a bar shape. Connected to the middle of the bar is a member of one side of the jointed element, and connected to the ends of the bar is a member of another side of the jointed element. The torsion bar has a relaxed state, setting an angle to which the joint will tend in the absence of external constraining forces. When the relaxed state is flat, the joint in the relaxed state will be flat (straight). When the relaxed state is twisted (*e.g*., because it was superelastically set to be twisted), the joint in the relaxed state will be torqued (e.g., bent outward, or in another direction). Forces that act to deform the joint from the relaxed state will be opposed by restorative forces. Torsion bars provide a potential advantage for storing a relatively large amount of potential energy in the spring for a given angular deformation (by twisting around a longitudinal axis), compared, for example, to deformation (deflection of the longitudinal axis) of a leaf spring of similar dimensions through the same angle.

In some embodiments, torsion bars enable manipulation of tissue between the expanded anchoring configuration and collapsed suturing configuration by a purely rotational movement. The movement is potentially without radial expansion. The movement is potentially along a direct, and potentially shortest route. This provides a potential advantage for reducing a likelihood of tissue disconnection from the anchors.

In some embodiments, torsion bars are angled at different angles relative to one another in order to provide arms of a suturing clip with a plurality of different corresponding angles of rotation. This is a potential advantage for increasing flexibility of configuration options in moving between a pre-movement configuration and a post-movement configuration of suturing clip components such as anchors.

An aspect of some embodiments of the present disclosure relates to shaping of an aperture in preparation for receiving a suturing clip. In some embodiments, an aperture spreader is actuated to engage with the sides of an aperture (such as an LAA ostium), in preparation for the advance of a suturing clip toward the aperture, expansion of the suturing clip, and/or anchoring of the suturing clip into tissue of the aperture.

Before explaining at least one embodiment of the present disclosure in detail, it is to be understood that the present disclosure is not necessarily limited in its application to the details of construction and the arrangement of the components and/or methods set forth in the following description and/or illustrated in the drawings. Features described in the current disclosure, including features of the invention, are capable of other embodiments or of being practiced or carried out in various ways.

### Left Atrial Appendage Closure by Suturing Clips

### Closure of the LAA

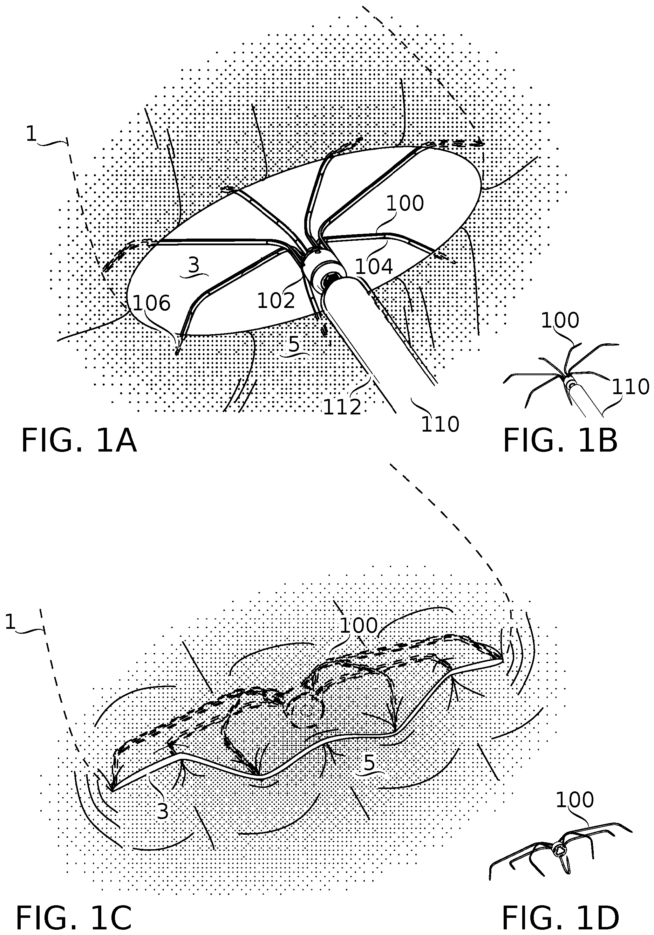

Reference is now made to *Figure 1A**,* which schematically illustrates a suturing clip **100** engaged to tissue within a LAA **1,** according to some embodiments of the present disclosure. Reference is also made to *Figure 1B**,* which schematically illustrates the suturing clip **100** of *Figure 1A* in an expanded configuration suitable for tissue engagement, according to some embodiments of the present disclosure. Further reference is made to *Figure 1C**,* which schematically illustrates a suturing clip **100** which has engaged tissue within an LAA **1** and then undergone a conformational change to close an LAA ostium **3,** according to some embodiments of the present disclosure. Reference is also made to *Figure 1D**,* which schematically illustrates the suturing clip **100** of in the LAA-closing conformation of *Figure 1C**,* according to some embodiments of the present disclosure.

Suturing clip **100** is optionally configured according to any of a range of forms and designs, more particular examples of which include suturing clips **300, 350, 400, 500,** and **800,** described herein. These more particular examples vary from one another in features such as numbers of arms, specifics of arm shape, specifics of anchor shape, specifics of core design, and type of closure. Additionally or alternatively, embodiments of suturing clip **100** encompass any suitable arrangement of anchoring positions. For example, in the expanded anchoring configuration, the anchors are optionally arranged in a circular, elliptical, oval, split-curve (arranged along two or more perimeter sections), split-line (arranged along two or more line segments), and/or open-sided perimeter configuration. In the collapsed suturing configuration, for example, the anchors are optionally arranged in a circular (*e.g*., collapsed toward a common center), linear, zigzag, or another aperture closing configuration.

Generic features of a suturing clip **100** introduced in the descriptions of *Figures 1A-1F* should be understood to apply to any of the more particular embodiments herein, modified as explained in their more particular descriptions.

*Figures 1A* and *1C* are drawn from a perspective on an atrial side of an LAA, looking at the LAA ostium **3,** which is an opening in atrial wall **5** leading into LAA **1.** *Figures 1A* and *1C* show selected phases in the closure of LAA **1** using suturing clip **100.**

*Figure 1A* shows suturing clip **100** expanded within LAA **1,** and *Figure 1B* shows the same expanded suturing clip **100** without illustrating the surrounding tissue. Arms **104** of suturing clip **100** each deploy radially from their respective bases, where they connect to core **102.** Anchors **106,** positioned on respective terminal ends (*i.e.,* ends opposite the ends attached to core **102;** also referred to herein as "free ends") of arms **100,** are configured to engage tissue from within LAA **1,** for example by piercing, hooking, and/or pinching. In *Figure 1A**,* core **102** of suture clip 100 is connected to delivery system **110.** Delivery system **110** comprises, in some embodiments, a catheter device, for example a percutaneous catheter device, of which distal catheter end **112** is illustrated. Previous to achieving the expanded position of *Figure 1A**,* suturing clip **100** was delivered by delivery system **110,** *e.g.,* enclosed within a lumen of distal catheter end **112** in a collapsed delivery configuration.

*Figure 1C* shows suturing clip **100** in a collapsed suturing configuration, still engaged with tissue of LAA **1,** and *Figure 1D* shows the same collapsed suturing configuration of suturing clip **100** without illustrating the surrounding tissue. In the collapsed suturing configuration, suturing clip **100** draws the LAA ostium **3** closed from its position within LAA, creating a band of closure (optionally a zigzag band of closure as illustrated). LAA ostium **3** is closed potentially without any exposure of suturing clip **100** along an atrial side of atrial wall **5.** For example, suturing clip **100** is entirely within the LAA **1** on the side of the closed LAA ostium **3,** and/or suturing clip **100** is unexposed to the lumenal contents (*e.g*., blood) on the atrial side of atrial wall **5.** This is a potential advantage for preventing device-induced thrombogenesis producing blood clots having access to the circulating blood pool. Attachment at the ostium **3** and/or adjacent to the ostium **3** is potentially advantageous over attachment at other positions within the LAA, insofar as tissue there may be thicker and/or stronger than tissue within the inner LAA.

### Device Positioning in Preparation for LAA Closure

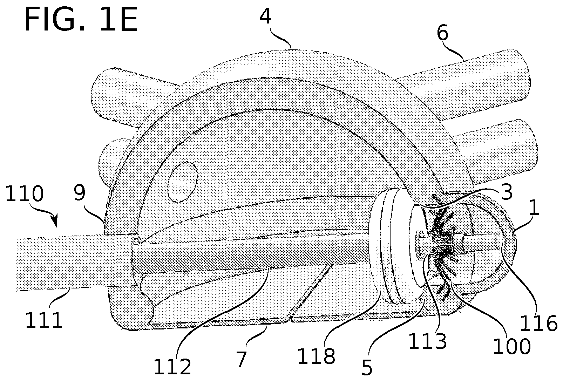

Reference is now made to *Figure 1E**,* which schematically illustrates a trans-septal approach insertion of a suturing clip **100** using a delivery system **110,** according to some embodiments of the present disclosure.

Delivery system **110,** in some embodiments, comprises a catheter overtube **111,** distal catheter end **112,** and delivery mount **113.** Optionally, there is provided to delivery system **110** a stopper **118;** configured, for example, as an inflatable balloon, expandable braid, and/or nitinol construction (for example, one or more flexible struts).

Also shown in *Figure 1E* are schematic representations of atrium **4,** including septal wall **9,** pulmonary veins **6,** mitral valve **7,** LAA **1,** and atrial wall **5** surrounding the ostium **3** of LAA **1.**

Suturing clip **100** is shown in an expanded anchoring configuration, wherein arms **100** press anchors **106** outward to positions contacting tissue of the ostium **3** of the LAA, whereat the anchors **106** can be manipulated (*e.g*., by movement of the suturing clip via delivery mount **113)** to anchor in the tissue.

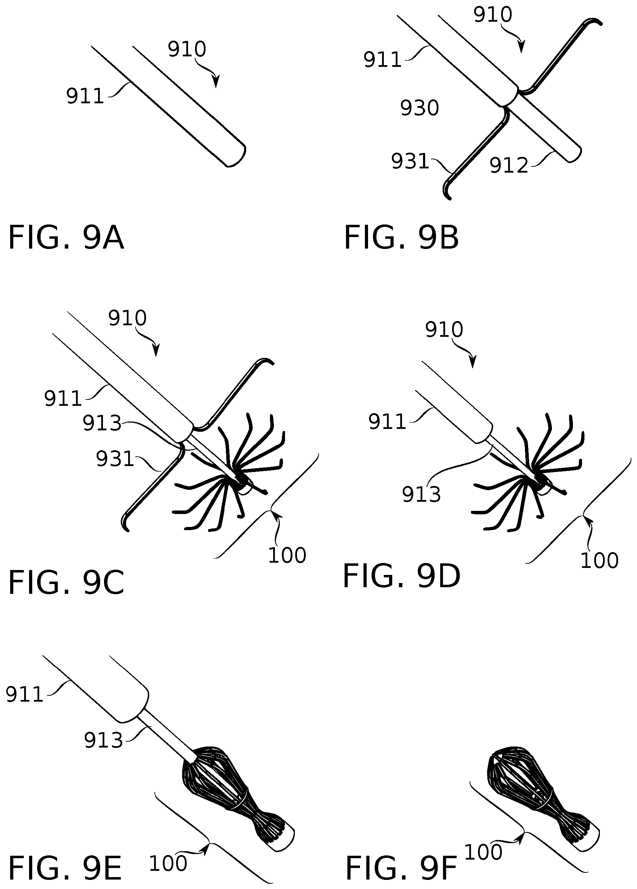

It should be noted that in some embodiments stopper **118** is replaced by and/or used together with an expander, for example expander **1000** as described in relation to *Figures 9A-10E.*

### Methods of LAA Closure

### Operations During LAA Closure

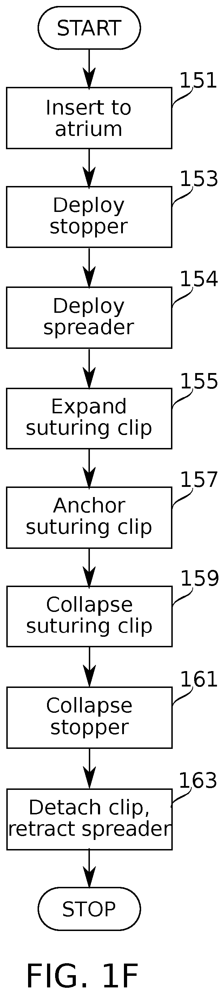

Reference is now made to *Figure 1F**,* which is a schematic flowchart of a method of inserting of a suturing clip **100** using a delivery system **110,** according to some embodiments of the present disclosure.

The flowchart of *Figure 1F* begins after transseptal access is gained to an atrium, for example using a 0.35 inch (8,89 mm) guidewire. The punctured septum is dilated, and the sheath of the suturing clip **100** (catheter distal end **112)** is introduced to the left atrium, and then positioned at the LAA ostium.

In some embodiments, deployment of a suturing clip **100** continues comprising the following operations:
At block **151,** in some embodiments, insert the suturing clip **100** to the left atrium using deployment system **110.**

At optional block **153,** in some embodiments, stopper **118** is deployed and positioned against atrial wall **5.** A tip **116** of deployment system **110** is positioned to pass into the ostium **3** of the LAA **1** when it is distally advanced. While stopper **118** is optional, it provides a potential advantage for ensuring that a correct (and preferably minimally required) insertion distance of suturing clip **100** is used during its deployment. Stopper **118** may also help to stabilize the LAA **1** and the delivery system **110** relative to one another during deployment.

At optional block **154,** in some embodiments, spreader **1000** (described in relation to *Figures 9A-10E)* is deployed, for example as described in relation to *Figures 9A-9F**.*

At block **155,** in some embodiments, the suturing clip **100** is deployed to an expanded anchoring position; *e.g*. at least partially unsheathed by pushing forward on a control member of deployment system **110** to move forward portions of the system which hold and control the suturing clip (also referred to herein as the "internal system", and comprising delivery mount **113).**

As suturing clip **100** advances out of distal catheter end **112,** it changes shape from its collapsed delivery configuration to its expanded anchoring configuration. In some embodiments, expansion is automatic, as the arms **104** expand to assume their superelastically set shape.

At block **157,** in some embodiments, the suturing clip **100** is anchored. In some embodiments, anchoring comprises pulling the suturing clip **100** backwards using the internal system, until it anchors into tissue of the ostium wall via anchors **106** positioned on the ends of arms **104.** Optionally, a position of suturing clip **100,** its arms **104** and/or its anchors **106** is verified before and/or after anchoring, for example by use of imaging observation (*e.g*., ultrasound imaging and/or fluoroscopy). Optionally, anchoring is verified by a "tug test", comprising one or both of detecting resistance to retraction, and viewing movement under imaging observation. Optionally, suturing clip **100** is provided with one or more radiopaque markers to assist in visualization of the anchors **106,** arms **104,** and/or core **102** of the device. For example, anchors **106** or another part of suturing clip **100** comprise an alloy more radiopaque than nitinol (*e.g.,* a radiopaque marker comprising gold, tungsten, or another material). In some embodiments, delivery system **110** comprises a radiopaque marker, positioned to identify, for example, a portion of delivery mount **113.**

At block **159,** in some embodiments, the suturing clip is collapsed to its collapsed suturing configuration. Developing sufficient closing force from the suturing clip **100** is a potential challenge. A typical catheter inner lumen diameter, for example, is about 3-5 mm, within which the collapsed delivery configuration of the suturing clip is positioned. The diameter of the LAA may be approximately 30 mm, potentially creating a situation of mechanical disadvantage, since by the principles of leverage, forces exerted tend to reduce as distance from the fulcrum increases.

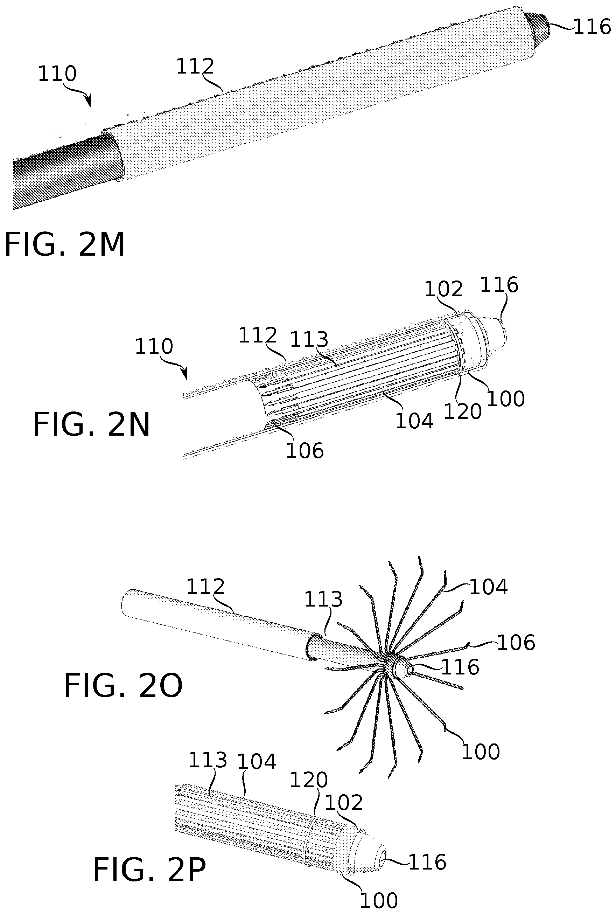

Closing force, in some embodiments, is exerted by using the internal system to pull backward on a closure disk **120** (*e.g.,* as described in relation to *Figures 2N-2P**,* herein). In some embodiments, a closure disk **120** operates by sliding along a plurality of arms from a base region of the arms and toward an anchoring end of the arms. The closure disk is connected to the arms (*e.g.,* via one or more apertures) so that as it moves, it changes radial expansion of the arms **104,** and thereby collapses the arrangement of anchors **106** into their collapsed suturing configuration, drawing anchoring tissue of the ostium **3** along with them. Closure disks are also referred to herein as closure "rings". Examples and additional description of closure disks, their functioning and their operation are also described, for example, in relation to *Figures 2M-4H**,* herein. Closure disks provide a potential advantage by bringing the fulcrum of mechanical leverage closer to the anchoring sites at which the tissue closure is formed.

Optionally, the roles of superelasticity and the disk are reversed. For example, the suturing clip, in some embodiments, is superelastically set to assume the closed position, and the disk initially set in a more proximal position. Movement of the disk forced *distally* potentially also forces the suturing clip to open and assume the expanded anchoring state, *e.g.,* by approaching a distal part of the suturing clip whereat the arms come together within a radius smaller than the apertures in the disk through which they pass. Upon relaxation of force on the disk, the arms tend to close, urging the disk proximally again. Optionally, the disk is pulled proximally again. It is noted that the self-expanding configuration of such disk-controlled devices is potentially more mobile than a corresponding self-collapsing configuration, due to differences in size constraints.

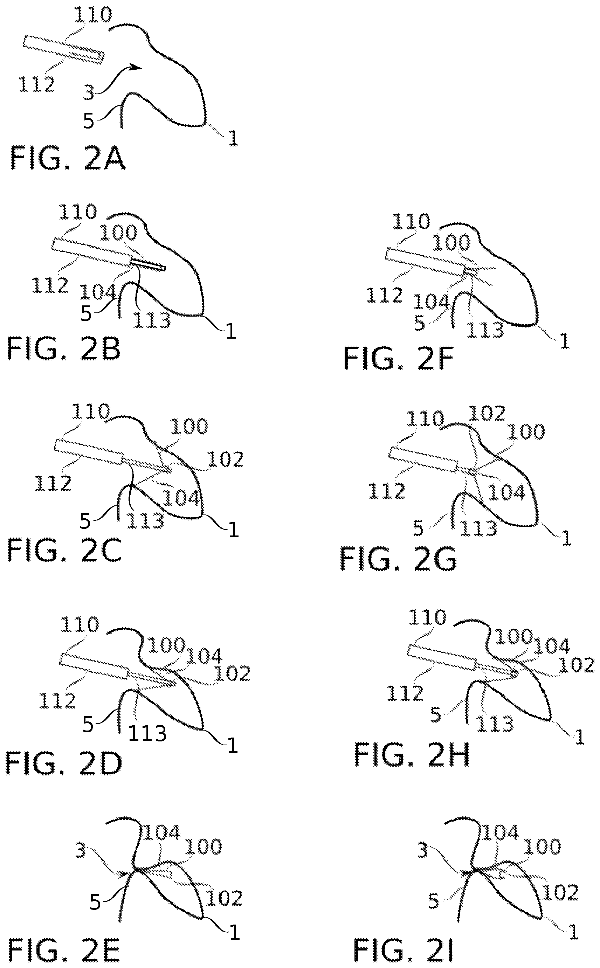

In some embodiments, another method of developing force for collapse to the collapsed suturing configuration is used; for example an everting-type closure, wherein the arms are themselves resiliently biased to continue on toward closure after passing through a more expanded configuration. In such configurations, the collapsed delivery configuration of arms **104** as contained by delivery system **110** may orient them to extending from a proximal side near core **102** distally toward their free anchoring ends. It may be noted that the design used as an example in *Figures 1A-1D* to illustrate certain generic features of a suturing clip **100** happens to undergo everting-type closure. The design used as an example in *Figure 1E* of certain generic features of a suturing clip **100** and its delivery system **110** happens to undergo what is referred to herein as reverting-type closure. Features related to the specifics of these closure types (as well as other details of the specific designs) are discussed in relation to the several more particular embodiments of the device, and, for example, in relation to *Figures 2A-2I**,* herein. In an everting design, leverage is potentially increased in part by the collapse of the arm itself to a shorter leverage distance due to curvatures along the length of the arm. Moreover, a portion of the force generated for closure is optionally created through use of a torsion bar feature (also referred to herein as a "torque beam") in the design of the suturing clip **100.** A torsion bar is a spring design (exerting rotational force through a plane transverse to a longitudinal axis of the bar or beam) which can potentially exert relatively high force compared to a leaf spring (*e.g*., flexible arm, exerting rotational force through a plane passing along a longitudinal axis of the arm) of the same general size and material.

In some embodiments, closure is verified; for example by fluoroscopy and/or ultrasound (echocardiography). In some embodiments, closure is verified by the tracking of dye from an injection made within or outside the LAA.

In some embodiments, closure is followed by suction and/or an injection of material (*e.g*., saline) to remove and/or displace blood remaining the LAA. This potentially helps reduce a possibility of a blood clot forming even within the closed-off pocket of the LAA formed by the suturing clip.

At optional block **161,** in some embodiments, stopper **118** (if used) is re-collapsed.

At block **163,** in some embodiments, the delivery system **110** is detached from the suturing clip **100** and removed from the body. Optionally, detachment occurs as part of the collapse to a suturing configuration of suturing clip **100,** and/or immediately thereafter.

Optionally, operations of the various blocks are returned to in reverse during deployment, for example in order to reposition the suturing clip **100** based, for example, on fluoroscopic observations (*e.g*., spread of injected dye), and/or other observations and/or feedback.

### Closure of an Aperture from a Side Opposite the Approach Side

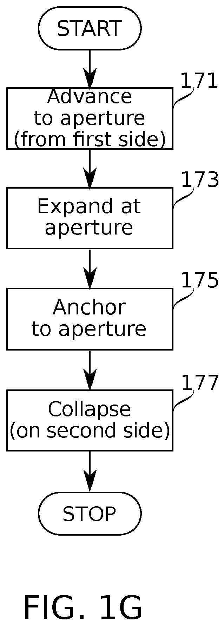

Reference is now made to *Figure 1G**,* which is a schematic flowchart of closure of a body lumen aperture by a suturing clip **100** which closes the aperture from a side of the closure opposite a side from which the suturing clip is introduced, according to some embodiments of the present disclosure.

At block **171,** in some embodiments, the suturing clip **100** is advanced from a first side of a body lumen aperture (*e.g*., an ostium **3** of an LAA **1)** toward a second side of the body lumen aperture.

At block **173,** in some embodiments, the suturing clip **100** is expanded with at least a portion of it positioned on the second side of the body lumen aperture.

At block **175,** in some embodiments, the suturing clip **100** is anchored into the body lumen aperture (*e.g*., using anchors positioned on the portion of the suturing clip **100** on the second side of the body lumen aperture).

At block **177,** in some embodiments, the suturing clip **100** is collapsed to close the body lumen aperture, with the suturing clip 100 remaining on the first side of the body lumen aperture.

### Clip Configuration Changes During LAA Closure

Reference is now made to *Figures 2A-2I**,* which schematically illustrate stages in the deployment of a suturing clip **100,** according to some embodiments of the present disclosure. Herein, the reference characters "100" are used in descriptions of suturing clips in general, with certain more particular embodiments being given their own reference characters identifying them as species of the genus, and/or described with reference to their particular characteristics.

*Figure 2A**,* in some embodiments, represents the approach of a delivery system **110** including a distal catheter end **112** toward an ostium **3** of a left atrial appendage **1** in the atrial wall **5** of a heart atrium.

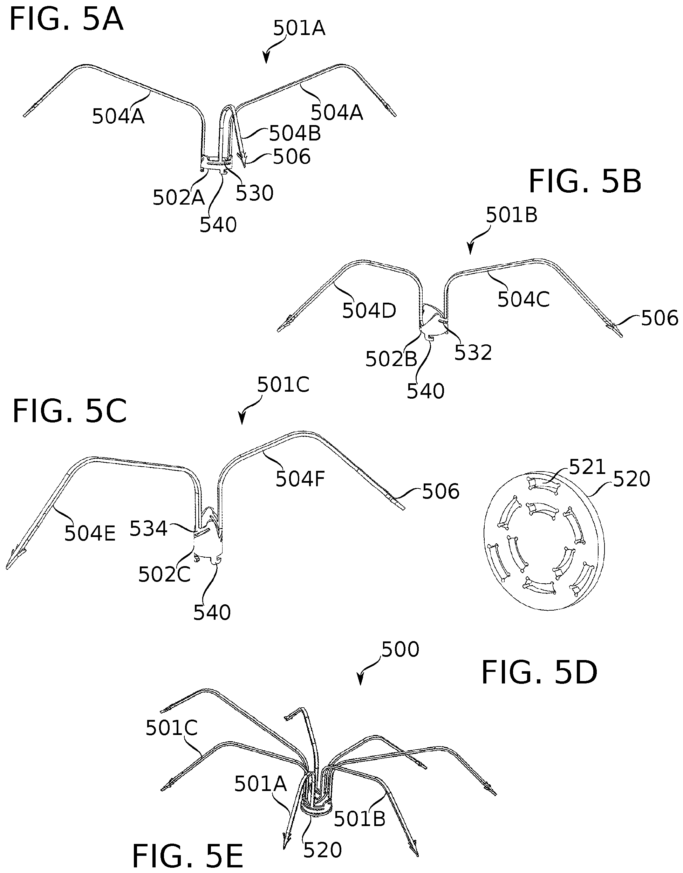

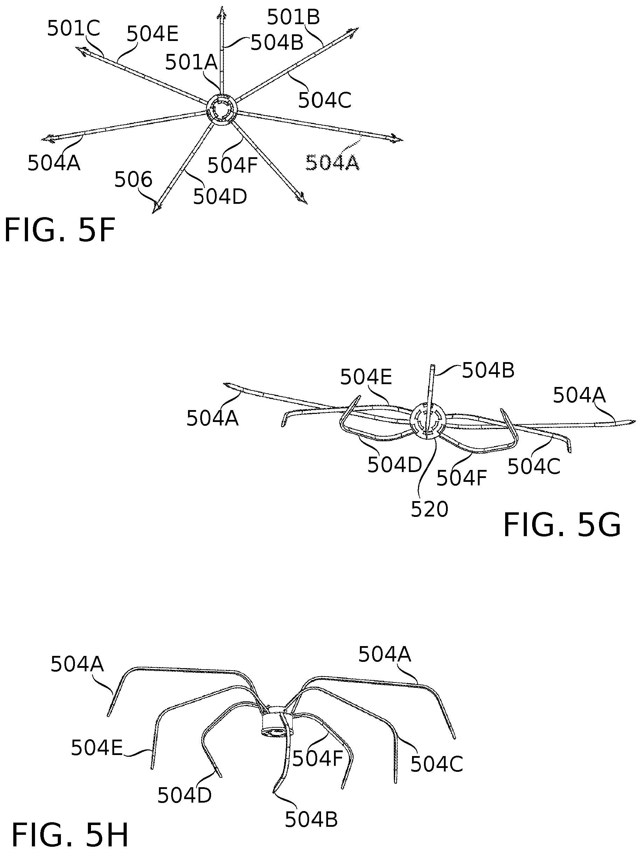

*Figures 2B* and *2F**,* in some embodiments, represent partial unsheathing of suturing clip **100** by actuation of delivery system **110.** Unsheathing comprises, for example, retraction proximally of distal catheter end **112** from delivery mount **113,** and/or extrusion distally of delivery mount **113** from catheter end **112.** In the example of *Figure 2B**,* suturing clip **100** is packaged with core **102** on a distal side of the collapsed delivery configuration, and anchoring ends of arms **104** on a proximal side of the collapsed delivery configuration. In the example of *Figure 2F**,* the configuration is reversed distal-proximally, for example, as described in relation to *Figures 5A-**8G,* herein. Anchoring ends on the more distal side is also the configuration shown in *Figures 1A-**1D.*

*Figures 2C* and *2G**,* in some embodiments, represent expansion of arms **104** of suturing clip **100** radially away from core **102** (relative to the positions of *Figures 2B* and *2F*, respectively); and their initial engagement (via anchors **106,** not illustrated) with a lumenal side of tissue within LAA **1.** Expansion of arms **104,** in some embodiments, comprises a superelastic configuration change due to arms **100** being set, when unconstrained, to expand as shown. Additionally or alternatively, a separately actuated mechanism forces expansion of arms **104.**

Comparing *Figures 2C* and *2G**,* it may be seen that there is a potential advantage of lowered initial insertion distance for an everting suturing clip as in *Figure 2G**.* Lowered insertion distance potentially reduces a risk of LAA perforation. This risk occurs, for example, since the LAA interior comprises relatively thin and/or fragile tissue compared to the muscular wall of the rest of the heart, and/or because of potential difficulties in obtaining a clear view of the complicated 3-D shape of the LAA. In contrast to inserting a whole suturing clip **100** in a still-collapsed (and thus longitudinally maximally extended) configuration (and then expanding), an everting-type closure suturing clip **100** can be gradually expanded as it advances distally, with the most-expanded part also being its distal-most portion. This potentially reduces a risk of perforation by elements positioned on the relatively stiff central axis of the delivery system, since these elements are not distal-most during an important stage of initial deployment, and optionally are not themselves inserted into the LAA during deployment, or inserted to a minimal degree. Insofar as the suturing clip **100** itself is "self-driving", it is the case in some embodiments that it need only be engaged at a proximal side of ostium **3** of LAA **1,** and not actively inserted further than this. Once collapse to the collapsed suturing configuration is allowed to proceed, it draws itself into the LAA, to a distance limited by the previously established anchoring fixation. It should be noted that non- or minimal-insertion deployment also potentially reduces a chance of dislodging a potentially pre-existing thrombus during the deployment procedure. After anchoring, there remains some protection given from potential perforation events, since the anchoring itself controls a maximal distance of delivery system advance (*e.g*., prevents further advancing).

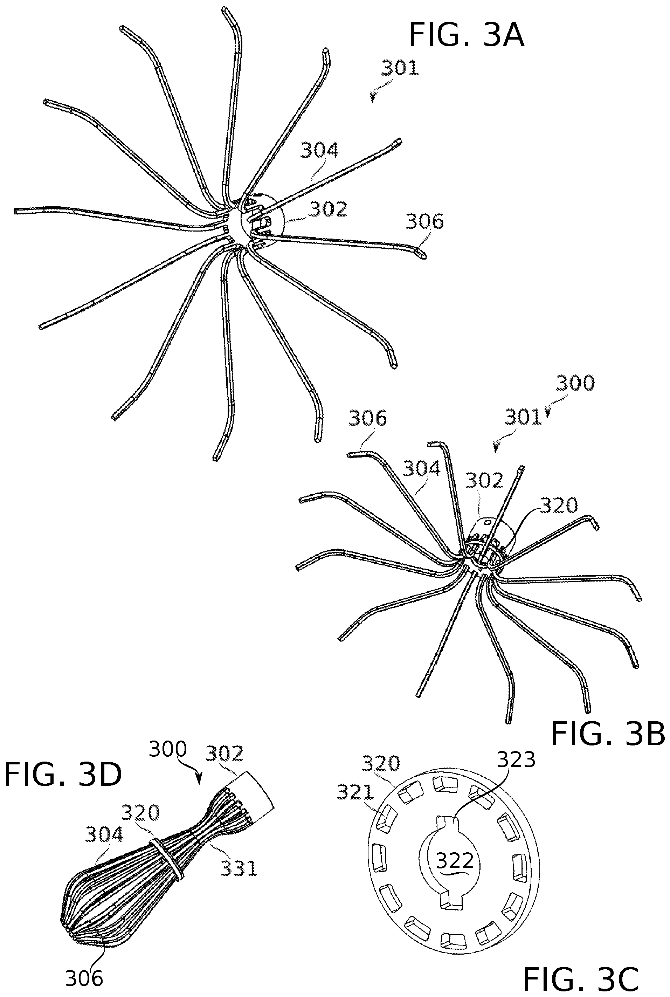

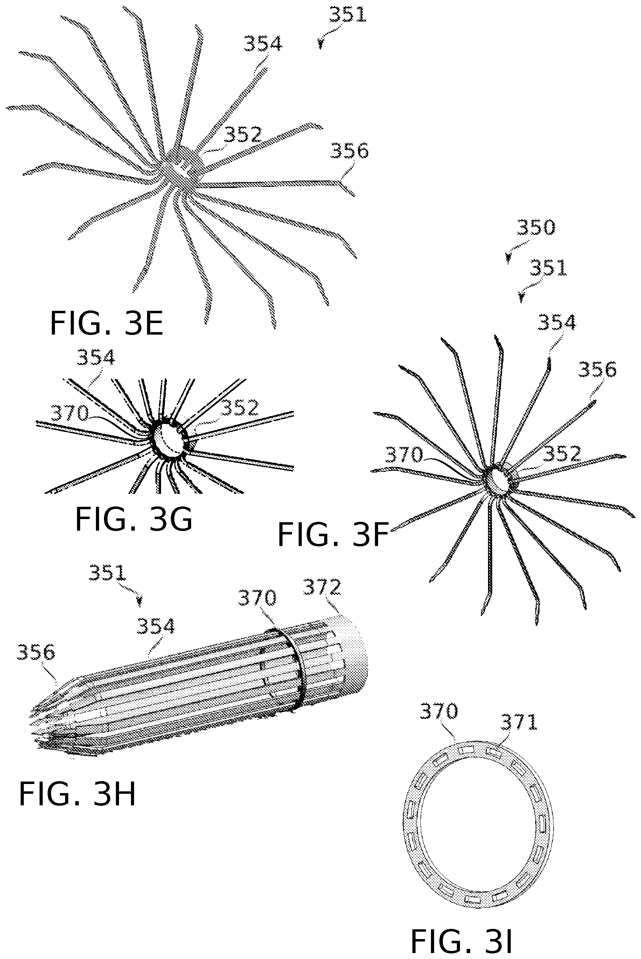

*Figures 2D* and *2H**,* in some embodiments, each represent an intermediate stage of closure of arms **104** toward a collapsed suturing configuration. Different actuation mechanisms and/or shape transitions may be used to complete the closure of ostium **3.** In *Figure 2D**,* for example, arms **104** are re-collapsing by being forced back along the same direction from which they expanded. Herein, this is also referred to as a "reverting"-type closure. Optionally, this is accomplished by movement of a closure disk **320** (*Figure 3C*) that slides proximally along arms **104,** forcing them closed. In some embodiments, additionally or alternatively, resilient elasticity of arms **104** is used to provide force for closure.

Optionally, closure comprises a continued conformational change of arms **104,** for example as shown in *Figure 2H**.* Herein, this type of closure is also referred to as an "everting" closure, wherein initially distally-pointing arms **104** (*e.g.,* oriented as in *Figure 2F*) continue to rotate past their most-expanded state, reversing their proximal-distal orientation relative to core **102** to point proximally. The suturing clip **100** of *Figures 1A-1D* also represents an "everting"-type suturing clip. A potential advantage of everting-type closure is a lowered distance of penetration into the LAA during insertion, reducing one or more of a risk of perforation and a risk of dislodging a pre-existing thrombus.

*Figures 2E* and *2I*, in some embodiments, each represent a final stage of closure, wherein the arms **104** have reached a fully closed collapsed suturing configuration. LAA ostium **3** is now closed. Delivery system **110** is detached, and suturing clip **100** remains attached to lumenal-side tissue of LAA **1,** and sequestered within LAA. Insofar as suturing clip **100** is so-sequestered, it is prevented from acting as a center of thrombogenesis for blood clots which could enter the general circulation. Potentially, the sutured sides of the ostium **3** of LAA **1** will gradually grow together, preventing re-opening of LAA **1.**

### Anchor Movements During LAA Closure

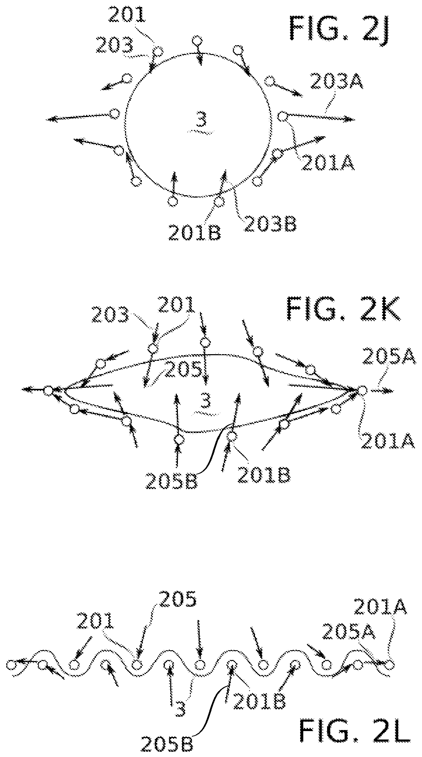

Reference is now made to *Figures 2J-2L**,* which schematically illustrate movements **203, 205, 203A, 203B, 205A, 205B** of anchoring positions **201, 201A, 201B** over the course of a transition of a suturing clip **100** between a deployed- and-anchored configuration, and a collapsed suturing configuration, according to some embodiments of the present disclosure. The movements are shown as they would be viewed from a direction looking into the LAA through the aperture of ostium **3.**

The movements shown optionally apply to either or both of everting and reverting suturing clips **100.** In *Figure 2J**,* anchoring positions **201** are initially arranged around an interior circumference of ostium **3.** Herein the reference characters **201** refer generically to anchoring positions (of which one particular instance is labeled), and the reference characters for anchoring position **201A** refer to a particularly labeled instance of an anchoring position **201.**

As a suturing clip **100** collapses (*Figure 2K*)*,* the anchoring ends of each arm **104** move in different directions within the viewing plane. Arrows **203, 205** (as identified generically) each point in a different direction, indicating the position to which each anchoring position **201** moves between the configurations of *Figure 2J* and the positions of *Figure 2K* and then *Figure 2L**.*

It is noted in particular that there is motion of some anchoring positions (*e.g*., anchoring position **201A** in directions **203A** and then **205A)** radially *outward,* while other anchoring positions (*e.g*., anchoring position **201B** in directions **203B** and then **205B)** move generally radially *inward.* There is, however, a convergence in common to a shared suturing line. As a result, suturing clip **100** gradually draws ostium **3** into a new, nearly linear shape which retains most of the perimeter length of original ostium **3** (*e.g.,* at least 50%, 70%, 80%, 90%, or 100% of the original perimeter length). Herein, the term "laterally-displacing collapse" is used to refer to collapsing movements of anchoring positions **201,** arms **104,** anchors **106,** and/or other elements of a suturing clip **100,** which act to stretch an aperture in one direction (*i.e.,* laterally) while collapsing it in another.

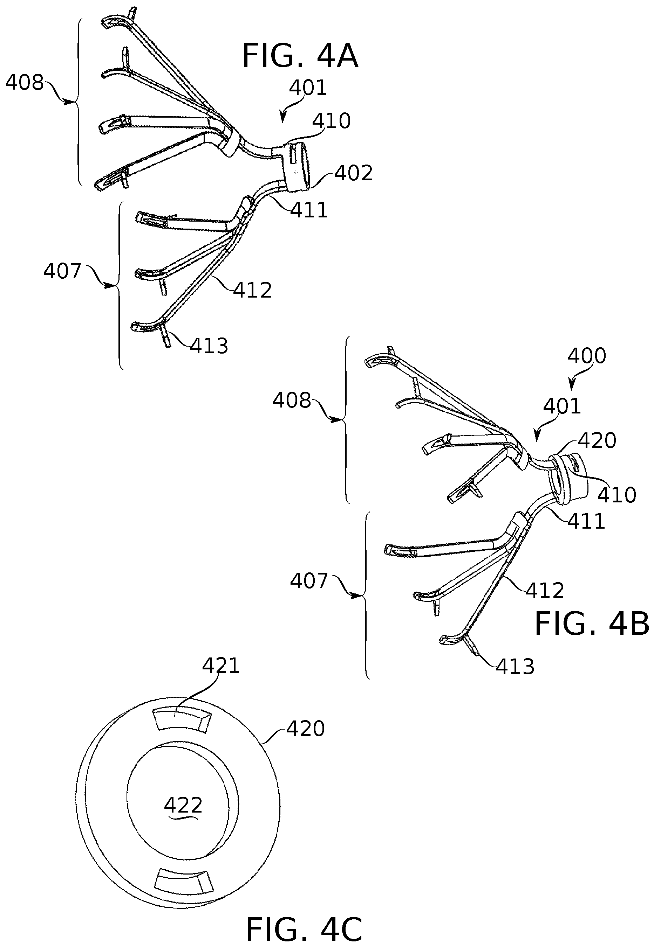

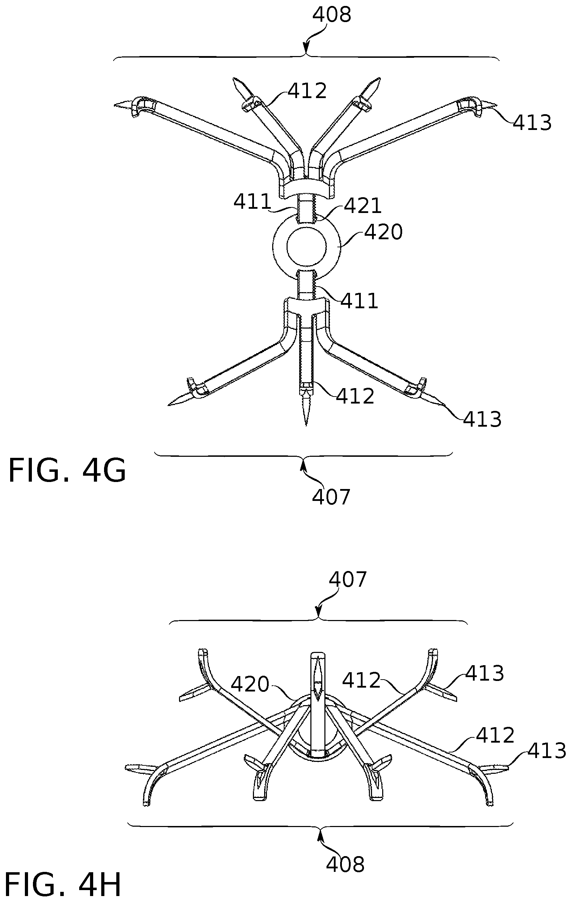

A pattern of laterally-displacing collapse is seen, for example, in the embodiments of *Figure 5A-8G**,* and in another form in the embodiments of *Figures 4A-4F**.* While not every embodiment of the current disclosure shows laterally-displacing collapse (*e.g.,* the embodiments of *Figures 3A-3D*)*,* laterally-displacing collapse it is a potential advantage to help reduce stresses due to "puckering" or "gathering" which could occur when all anchoring positions are drawn toward a common radial center.

*Figure 2L* shows the anchoring positions **201** in the collapsed suturing configuration. The actual collapsed suturing configuration depends on a balance between forces placed on anchoring positions **201** urge them toward the same line-or even toward positions *past* the same line-and forces that resist this motion. In some embodiments, the result is a wavy ("clam shell") or other irregular pattern along the lip of ostium **3.** For example, tissue near to an anchoring position **201** tends to press further inward, while tissue further away from an anchoring position tends to give way to this pressure. To this extent, mechanical properties of the tissue itself become part of the seal created by the suturing clip **100** at the site of the ostium **3.** In the collapsed suturing configuration, for example, the anchors **106** are optionally arranged in any suitable configuration for closure, *e.g.,* a circular (*e.g.,* collapsed toward a common center), linear, zigzag, or another aperture-closing configuration.

### Delivery Packaging and Closure Ring Operation

Reference is now made to *Figures 2M-2P**,* which illustrate details of interactions between a delivery system **110** and a suturing clip **100,** according to some embodiments of the present disclosure.

*Figure 2M* shows a delivery system **110** in a pre-deployment configuration. Suturing clip **100** is held within distal catheter end **112** in a collapsed delivery configuration. Optionally, distal catheter end **112** comprises a sheath of tubing at least long enough to fit over collapsed suturing clip 100. It may be as long as the whole catheter, or it may comprise a shorter segment which is actuated to withdraw by a control member such as a rod or wire controlled from a proximal side of the delivery system **110,** outside the body. Optionally, catheter distal tip **116** comprises an atraumatic tip, for example, constructed of a suitable polymer material.

In *Figure 2N**,* distal catheter end **112** has been rendered transparent, in order to show details of suturing clip **100** in its collapsed delivery configuration. In the example shown, arms **104** are held flattened against delivery mount **113,** constrained by being held within distal catheter end **112.** Anchors **106** occupy a proximal side of the arms **104** in this configuration, and core **102** occupies a distal side of the arms **104.** In some embodiments, closure disk **120** is initially positioned near the base of the arms at core **102.** In this position, closure disk **120** exerts little or no control over the expansion of arms **104** when they are extruded from catheter distal end **120** (and/or catheter distal end **120** is withdrawn), for example as shown in *Figure 2O**.*

In *Figure 2O*, the arms **104** are shown expanded, positioning the anchors **106** in a radially symmetrical pattern. Optionally, arms **104** expand to place anchors **106** in another configuration, for example, a pattern corresponding to portions of a perimeter of the ostium **3** of an LAA. Arms **104** expand enough to bring anchors **106** in contact with tissue. Arms **104** are potentially prevented from fully expanding to assume their superelastically set shape by this contact, and in this condition forces developed in the arms **104** themselves may assist in achieving tissue anchoring. Optionally, the suturing clip **100** is manipulated (*e.g*., pulled proximally and/or rotated using delivery mount **113)** to achieve tissue anchoring.

In *Figure 2P**,* suturing clip **100** is shown closed again, this time to its collapsed suturing configuration. In this case, the collapse comprises a proximal movement of closure disk **120** relative to core **102.** This proximal movement may be obtained using delivery mount **113,** since once anchors **106** are anchored to tissue, the rest of the device will tend to resist distal movement.

Optionally, the internal system comprises a separately controllable member for moving closure disk **120,** while delivery mount **113** remains in position. This provides a potential advantage, for example, for reversibility of deployment. Using such a control configuration, suturing clip **100** is optionally completely re-collapsed so that it can be once again withdrawn into catheter distal end **112.**

### Suturing Clip with Radial Arms and Closure Disk

Reference is now made to *Figure 3A**,* which schematically illustrates an expanded configuration of a reverting core-and-arm assembly **301** of a suturing clip **300,** according to some embodiments of the present disclosure. Reference is also made to *Figure 3B**,* which schematically illustrates suturing clip **300** an expanded configuration, including closure disk **320,** according to some embodiments of the present disclosure. Further reference is made to *Figure 3C**,* which schematically illustrates suturing clip **300** in a re-collapsed suturing configuration, collapsed by movement of closure disk **320,** according to some embodiments of the present disclosure, and additional reference is made to *Figure 3D**,* which schematically illustrates suturing closure disk **320,** according to some embodiments of the present disclosure. Suturing clip **300** is an example of a reverting-type suturing clip **100.**

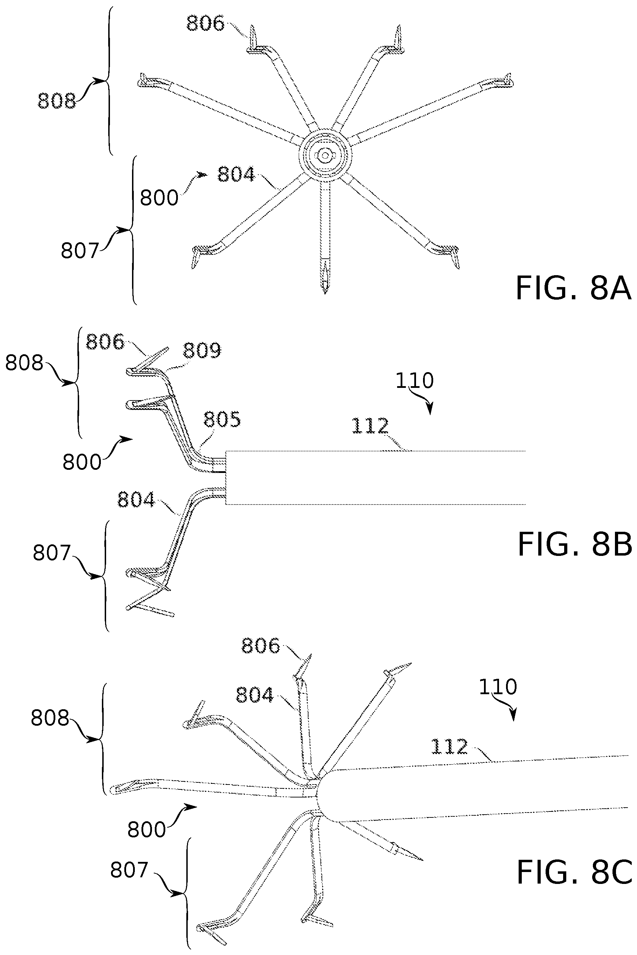

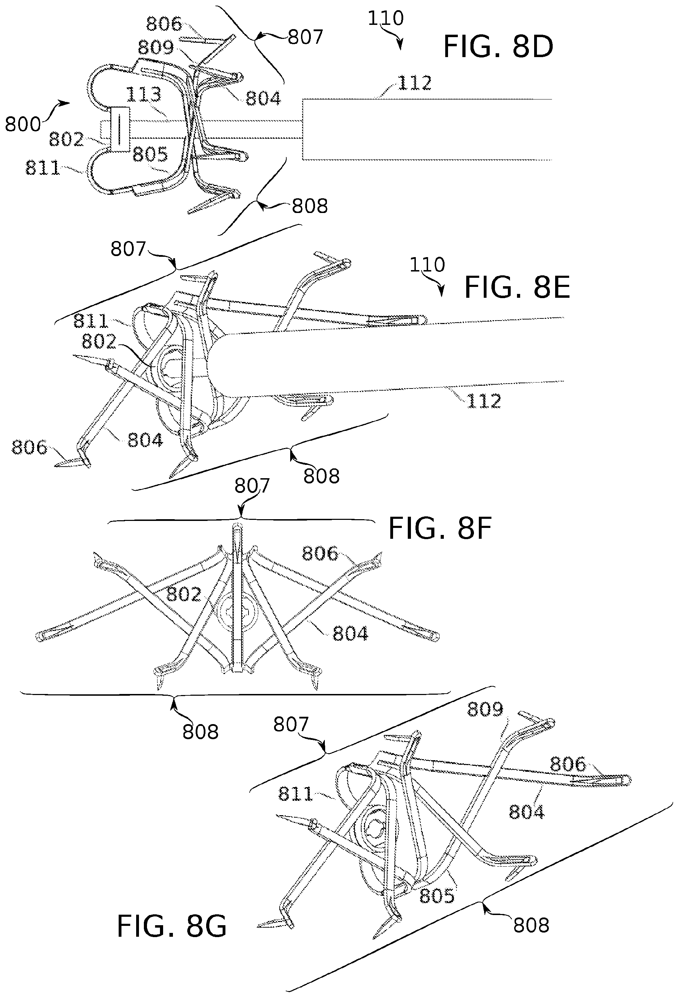

In some embodiments, a suturing clip **300** comprises a core-and-arm assembly **301** (*Figure 3A**)* and a closure disk **320** (*Figure 2C*)*,* assembled together so that arms **304** of core-and-arm assembly **301** pass through apertures **321** of closure disk **320.** Prior to delivery, arms **304** are collapsed and held in place, *e.g.,* by a surrounding lumen of a portion of delivery system **110.** As the arms **304** are released from this constraint, they expand toward the arm position shown in *Figure 3B**.* Constraining ring **320** at this stage is positioned at the base of arms **304,** near core **302.** The expanded device is manipulated within an LAA **1** so that anchors **306** anchor (fasten) into tissue. Details of anchors **306** are suppressed in the schematic drawing, but can be of any design suitable for piercing and/or gripping tissue, for example spiked anchor **413** of *Figure 4A**,* spiked anchor **806** of *Figure 8A**,* and/or barbed anchor **506** of *Figure 5A**.*

Once the anchors are attached, closure disk **320** is moved from its initial position near core **302** toward the anchoring ends of the arms **304.** This acts to pull the arms **304** inward, so that their tips converge. Accordingly, tissue surrounding a closure created by a device of this design tends to form a radial "pucker" or surgical purse-string result, as tissue is forced into a smaller region than its natural perimeter allows. Mechanical strength to do so is obtained from the increased stiffness of the arms as they shorten, and the strength and mechanical advantage of closure disk **320.**

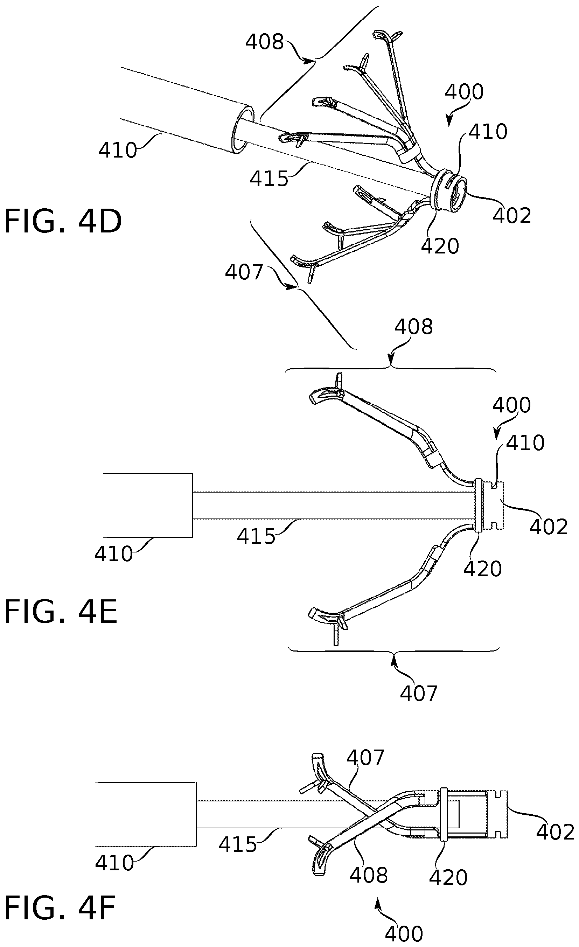

Optionally, a single deployment member acts both to carry suturing clip **300** into position and to pull closure disk proximally, since the force of anchoring will tend to resist the force of pulling the single deployment member proximally (this is further described, for example, in relation to *Figures 4E-4F* and suturing clip **400).**

Alternatively, movement of closure disk **320** relative to base **302** is optionally obtained by mounting the whole suturing clip **300** to a first deployment member of delivery system **110,** and then using a second deployment member to pull closure disk proximally while the rest of the device is held in place by the first deployment member.

In some embodiments, closure disk **320** comprises a locking aperture **322** through which the first and optionally the second deployment member of delivery system **110** extend. In some embodiments, cutouts **323** of locking aperture **322** provide a passage to which a deployment member can be rotated to allow withdrawal after the device achieves closure.

Reference is now made to *Figure 3E**,* which schematically illustrates an expanded configuration of a reverting core-and-arm assembly **351** of a suturing clip **350,** according to some embodiments of the present disclosure. Reference is also made to *Figures 3F-3G**,* which schematically illustrate suturing clip **350** an expanded configuration, including closure disk **370,** according to some embodiments of the present disclosure. Further reference is made to *Figure 3H**,* which schematically illustrates suturing clip **350** in a re-collapsed suturing configuration, collapsed by movement of closure disk **370,** according to some embodiments of the present disclosure, and additional reference is made to *Figure 3I**,* which schematically illustrates suturing closure disk **370,** according to some embodiments of the present disclosure. Suturing clip **350** is an example of a reverting-type suturing clip **100.**

In terms of its use and mechanisms of action, suturing clip **350** is of the same family of reverting-type closure designs as suturing clip **300,** with, *e.g.,* a different number of arms, a different closed shape to the arms, and an explicitly illustrated shape (barbed) for anchors **356.** It may be noted that arms **304** collapse at a place along their length more nearly to a radial center (*e*.*g*.*,* at pinch **331** *Figure 3D*) than the arms of suturing clip **350.** This more radially pinched shape provides a potential advantage for generating additional inward force during closure, albeit closure disk **320** potentially provides the principle fulcrum once closure is completed. The pinched shape may also allow a different range of options for angles at which anchors **106** (of which, *e*.*g*., anchors **306, 356** are particular examples) contact tissue.

### Suturing Clip with Branched Arms and Closure Disk

Reference is now made to *Figure 4A**,* which schematically illustrate an expanded configuration of a core-and-arm assembly **401** of a suturing clip **400,** according to some embodiments of the present disclosure. Reference is also made to *Figure 4B**,* which schematically illustrates suturing clip **400** an expanded configuration, including closure disk **420,** according to some embodiments of the present disclosure. Further reference is made to *Figure 4C**,* which schematically illustrates suturing closure disk **420,** according to some embodiments of the present disclosure. Suturing clip **400** is an example of a reverting-type suturing clip **100.**

Like suturing clip **300,** suturing clip **400** comprises a core-and-arm assembly **401** and a closure disk **420,** wherein movement of closure disk **420** away from the core **402** and toward the tips of arm subassemblies **408, 407** brings about reverting collapse of the arm subassemblies **407, 408,** which otherwise tend bend and to expand the suturing clip radially upon release from enclosure during delivery.

Each of the arm subassemblies **407, 408** comprises a plurality of arms **412,** individually equipped with anchors **413** of the spike design shown, and/or of another anchor design. Arms **412** of each arm subassembly **407, 408** are biased to expand so that they spread their anchoring ends to define a partial perimeter. The partial perimeter may be, for example, linear, or with only a slight curvature (this is shown, for example, in the distal-to-proximal view of *Figure 4G*)*.* Arm assembly trunk **411,** for its part, is biased to bend when unconstrained so that the two sets of arms take up positions on either side of the ostium of the LAA.

Closure of suturing clip **300,** in some embodiments, comprises constraining the arm assembly trunks **411** to revert to more-straightened positions (*e.g*., by movement of closure disk **420** to slide along the arm assembly trunks **411).** The individual arms **412** are optionally allowed to remain spread out, so that the closure of the LAA is spread out into a flattened configuration.

Further reference is now made to *Figures 4D-4H*, which show deployment of suturing clip **400** under control of a deployment system **410,** including deployment member **415,** according to some embodiments of the present disclosure. In some embodiments, deployment member **415** is interconnected to the device through aperture **422** of closure disk **420.** In some embodiments, protrusions on deployment member **415** interlock with a portion of core **402** *(e.g.,* protrude into apertures **410).** If initially attached to core **402,** release of deployment **415** may be achieved, for example, by rotation of deployment member **415.**

Once suturing clip **400** is anchored, pulling deployment member **415** proximally moves closure disk **420** proximally along with it. As shown in *Figures 4F* (from the side) and **4G** (looking proximally), arm subassemblies **407, 408** are brought together by this movement. Optionally, they are shaped so that in a completely actuated position arm subassemblies **407, 408** interdigitate. Interdigitation optionally comprises movements of portions of the arms **412** on arm subassembly **407** from a first side of the arms **412** of arm subassembly **408** to a second side of the arms **412** of arm subassembly **408.** Connection of the anchors **406** to tissue may restrict the degree of interdigitation. The resulting closure may resemble the zigzag closure band of the ostium **3** in *Figure 1C**,* for example; extending alternately between arms **412** of arm subassembly **408** and of arm subassembly **407.**

Full release of deployment member **415** is optionally performed by partial rotation, stronger pulling, actuation of unlocking members on deployment **415,** and/or another method.

It should be noted that stages of deployment (for this and optionally any other embodiment of the present disclosure) are optionally fully- or partially-reversible. For suturing clip **400,** for example, this may be achieved by reconnecting deployment member **415** and suturing clip **400,** restoring closure disk **420** to its original position, and/or moving the re-expanded suturing clip **400** to extract it from its anchored position. Reversibility is a potential advantage during implantation (*e.g.,* to allow adjustments), and/or to facilitate an option for emergency retrieval of the device.

### Suturing Clip with Self-Collapsing, Radial Arms

Reference is now made to *Figures 5A-5D**,* which schematically illustrate parts of an everting suturing clip **500,** according to some embodiments of the present disclosure. Suturing clip **500** is an example of an everting-type suturing clip **100.**

In some embodiments, a suturing clip **500** comprises a plurality of arm subassemblies **501A, 501B, 501C** (collectively, arm subassemblies **501A-501C)** which each separately provide portions of both the core (*e.g.,* core portions **502A, 502B, 502C,** referred to collectively as cores **502A-502C** and/or equivalently in a unit as core **502)** and the arms (*e.g.,* arms **504A, 504B, 504C, 504D, 504E,** and **504F;** referred to collectively and equivalently as arms **504A-504F** and/or arms **504).** Arms **504A-504F** are optionally of a plurality of individual designs, having lengths, orientations, and/or bending behaviors which function collectively to achieve a targeted closure configuration. Arms **504A-504F** are each terminated by an anchor **506;** for example of the barbed anchor type shown, and/or of another design and/or type. In some embodiments a portion of the bending behavior of an arm **504A-504F** is determined by the orientation and preset (*i.e.,* superelastically set) rotation of a torsion bar by which it is attached to the core, for example, one of torsion bars **530, 532, 534** (also referred to collectively as torsion bars **530),** which are optionally oriented to rotate in different planes for different arms **504A-504F.**

In some embodiments, the arm subassemblies **501A-501C** are attached to one another via connecting disk **520,** *e.g.,* by insertion of hooks **540** of each arm subassembly **501A-501C** into apertures **521** of connecting disk **520.** Optionally, connecting disk **520** is secured to arm subassemblies **501A-501C** by glue and/or welding. Optionally, connecting disk **520** is of titanium or another material (*e.g*., another biocompatible alloy such as stainless steel), and the various arm subassemblies **501A-501C** are formed from a superelastic alloy such as nitinol.

Reference is now made to *Figures 5E-5F**,* which schematically illustrate assembled suturing clip **500** in an expanded configuration, according to some embodiments of the present disclosure.

The assembled suturing clip **500** shown in *Figures 5E-5F* appears in its extended state (which also happens to correspond, for example, to the state shown in *Figure 1A*)*.* From *Figure 5F* in particular, it can be seen that anchors **506** are arrayed around an oval and/or elliptical perimeter. Optionally, the perimeter can be selected to be any suitable shape for anchoring.

During deployment, this state is maintained by holding the bases of the arms straight by a constraining device (such as a surrounding lumen of deployment system **110).** The unconstrained anchoring ends of the arms are predisposed (*e.g*., superelastically set) to assume the positions shown, starting from a constrained position, wherein all of the arms **504A-504F** are collapsed (*e.g.,* by containment within a lumen) to extend along the same longitudinal direction as is shown for their bases. Thus, deployment begins with suturing clip in a first, collapsed delivery position, and proceeds to a second, expanded anchoring position.

In the extended state, suturing clip **500** is in a configuration suitable for establishing anchoring with tissue, for example corresponding to *Figure 2G**.*

Reference is now made to *Figures 5G-5H**,* which schematically illustrate assembled suturing clip **500** in an everted configuration, according to some embodiments of the present disclosure. Upon removal of constraints on movement of the bases of arms **504A-504F,** the arms continue their rotations (largely impelled, in some embodiments, by torsion of torsion bars **530, 532, 534),** moving from the expanded anchoring position of *Figures 5E-5F* into the collapsed suturing configuration of *Figures 5G-5H**.* In this configuration, the arms are everted, to the extent that their anchors **506** end up positioned on the side of the core parts (*e.g*., connecting disk **520)** opposite that on which they started. Illustration of barbs **506** has been suppressed in *Figures 5G-**5H.*

It may also be noted that there is again a "zigzag" suturing band established by arms **104A-104F,** as has also been described in relation to other embodiments herein. In the illustrated orientation of suturing clip **500,** the anchoring end of arm **504B** crosses from top to bottom, as do the anchoring ends of arms **504B** and **504E.** They interdigitate with arms **504D, 504F** (which cross from bottom to top), and with arms **504A** on either side, which also cross upward.

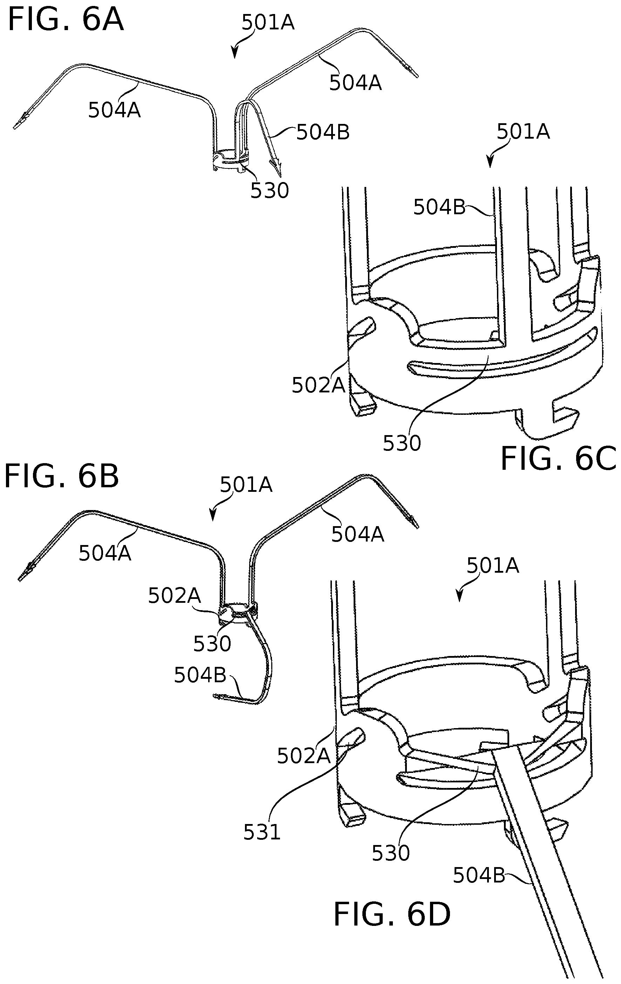

Reference is now made to *Figures 6A-6D**,* which schematically illustrate torsion bar arm mountings, according to some embodiments of the present disclosure. In some embodiments, any of arms **504A-504F** (and optionally any arm of any other embodiment described herein) connects to the suturing clip core through a torsion bar, *e.g.,* torsion bar **530.** Each of *Figures 6A-6D* shows just a single arm subassembly **501A,** with two of the arms **504A** fixed in their expanded anchoring configurations in order to emphasize movements of arm **504B** due to twisting of torsion bar **530.** In *Figure 6A* and the detail view of *Figure 6C**,* torsion bar **530** is shown in its constrained position. Once constraint is removed, torsion bar **530** is free to twist arm **504B** so that it ends up underneath core region **502A,** as shown in *Figure 6B* and in detail in *Figure 6D**.* The twisting potentially achieves a large relative positional shift of the anchoring end of arm **504B,** even if arm **504B** itself does not undergo any further conformational changes of its own when left unconstrained.

Torsion bar **530** (and the other torsion bars) provide a potential advantage for achieving reliable closure, insofar as torsion bars in a superelastic alloy are capable of exerting considerable force. It should also be noted that different torsion bars (*e.g*., comparing torsion bars **530, 531)** can be oriented to move arms through rotational movements in different planes. This provides a potential advantage for supporting the three-configuration deployment series which proceeds from the first, collapsed delivery configuration, through to the second, expanded anchoring configuration, and finally to a third, collapsed suturing configuration. Each configuration is optionally shaped to place the anchoring ends of the arms in different positions, not only with respect to the core, but also with respect to each other. For example, the anchoring ends of the arms move from a perimeter-like configuration in the expanded anchoring configuration to an interdigitated configuration in the collapsed suturing configuration.

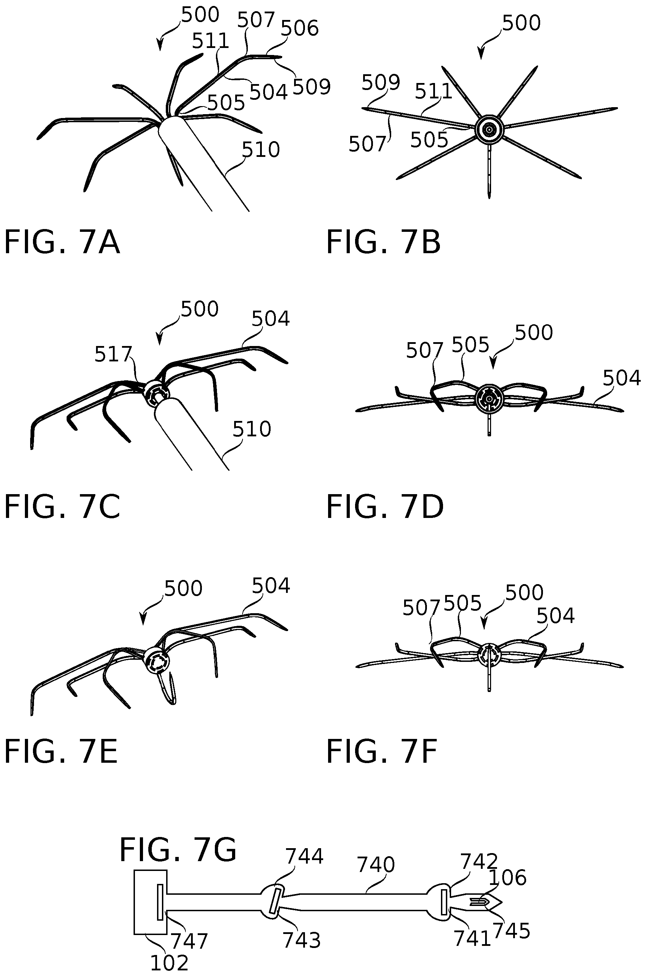

Reference is now made to *Figures 7A-7F**,* which schematically illustrate suturing clip **500** in three different stages of deployment, each from two different viewing angles, according to some embodiments of the present disclosure.

*Figures 7A-7B* show suturing clip **500** in the expanded anchoring configuration, from a perspective view (*Figure 7A*) and a distal-to-proximal view (*Figure 7B*)*.* In this configuration, each arm optionally comprises a pair of bends; basal bend **505** near the core **502,** and anchor bend **507** nearer to anchoring end of the arms. Optionally, this creates for each arm an anchoring segment **509** (carrying an anchor **506),** a middle segment **511** between the two bends **505, 507,** and optionally a base segment **517** which is basal on arm **504** relative to basal bend **505.** Optionally, each of bends **505, 507** bends within a shared plane for a given arm **504.** Moreover, for example as can be seen from the view of *Figure 7B**,* the bends **505, 507** are optionally within a multiplicity of planes sharing a common axis (as shown, the axis of the radial center of a distal portion of the delivery system **110).** This tends to orient the anchors **106** so that they face radially outward for contacting tissue of ostium **3.**

While these properties of bending direction potentially yield a convenient configuration for simplicity of manufacture and/or effectiveness of anchoring, embodiments of the present disclosure are not limited to have these properties. For example, bends **505, 507** of an arm **504** optionally bend in different planes, *e.g.,* due to a twist in the superelastically set shape at some point along intermediate segment **511.** Also optionally, bends **505, 507** of different arms **504** bend in a multiplicity of planes lacking a common axis.

*Figures 7C-7D* show suturing clip **500** in the collapsed suturing configuration, from a perspective view (*Figure 7C*) and a distal-to-proximal view (*Figure 7D*), with the delivery system **110** still attached. *Figures 7E-7F* show suturing clip **500** in the same collapsed suturing configuration, from a perspective view (*Figure 7E*) and a distal-to-proximal view (*Figure 7F*), with the delivery system **110** detached.

It may be noted that now the bends **505, 507** can be seen for some of the arms **504** as having shifted in their planar orientation. In the embodiment shown, this is achieved by twisting of torsion bars **530,** which are themselves longitudinally oriented in different directions. For example, some torsion bars **530** optionally extend longitudinally parallel to a plane of connecting disk **520.** Others (*e.g.,* as illustrated for torsion bars **532, 534)** extend obliquely to connecting disk **520;** *e.g.,* obliquely at an angle of about 20°, 30°, 45°, or another angle.

The number of degrees of freedom available for the multiply-jointed arms has a potential advantage in allowing the positions of anchors **506** in the expanded anchoring configuration to be decoupled from positions of the same anchors **506** in the collapsed suturing configuration. For each configuration and its functions, an optimal pattern of anchor positions can be determined, motion through the degrees of freedom available is adjusted correspondingly in the design and manufacture of the joints to create a suturing clip which transitions automatically between the determined patterns of anchor positions.

The collapsing motion to reach the suturing configuration is optionally isolated in time from motion to reach the expanded anchoring configuration of *Figures 7A-7B* by initially retaining constraint (*e.g.*, by distal catheter end **112)** on just the core **502,** after retraction of the constraint proximally past bends **507** and then bends **505.** Then, further proximal retraction removes the constraint on motion, allowing the torsion bars **530** to actuate. Motion through bends **507** and **505** can be similarly separated in time, optionally producing three distinct stages of deployment: a first bending to spread the anchoring segments **509,** a second bending to produce the deployed anchoring configuration of *Figures 7A-7B**,* and a third bending to produce the collapsed suturing configuration of *Figures 7C-7F**.* Correspondingly, there may be said to exist three "joints" per arm **504-**bends **505, 507** along the arm **504,** and torsion bar **530.**

It should be noted, moreover, that the motions can also be sequentially reversed by advancing constraint (*e.g.*, by distal catheter end **112)** again back over the arms **104.**

It may readily be understood that another number of joints per arm of a suturing clip **100** may be provided, in some embodiments. The joints are optionally shaped (*e.g*., superelastically set) to provide any suitable combination of planar and torsion movements to assume a plurality (optionally a multiplicity) of well-defined configurations at initial, final, and/or intermediate stages of deployment.

Reference is now made to *Figure 7G**,* which schematically illustrates a portion of a suturing clip **100** (only one arm is shown) comprising an arm **740** attached to a core **102,** in which bending of arm **740** is around one or more torsion bars **741, 743, 747,** including torsion bars **741, 743** which are positioned along the longitudinal extent of the arm **740.** The torsion bars **741, 743** are optionally created by cutting the bar shape from "elbows" **742, 744** of the arm **740.** As for torsion bars **530, 532, 534,** the torsion bar angle can be adjusted to change the plane of bending and/or final orientation of segments of arm **740.** Also shown is anchor cutout **745,** which illustrates a method of manufacturing an anchor **106-**the arm blank is cut (*e.g.,* laser-cut) with a slit extending on either side of a central spike (which optionally forms the anchor **106).** The spike is superelastically set to protrude from the arm tip when unconstrained, and flattened to lie within the arm **740** otherwise. It should be understood that arms **740** using the torsion bar elbow **742** are optionally used in place of any arm **104** of another suturing clip described herein, for example, in place of any arm **504.**

### Suturing Clips with Self-Collapsing, Branched Arms

Reference is now made to *Figures 8A-8G**,* which schematically illustrate everting-type closure suturing clip **800** comprising arm subassemblies **807, 808,** according to some embodiments of the present disclosure. Suturing clip **800** is an embodiment of a suturing clip **100.**

Suturing clip **800** comprises arm subassemblies **807, 808,** each comprising a plurality of arms **804** joined through an arm assembly trunk **811** to core **802.**

Bends in arms **805, 809** are exposed by withdrawal of constraint from catheter distal end **112** of delivery system **810,** allowing expansion of suturing clip **800** to an expanded anchoring configuration. The expanded anchoring configuration is in *Figures 8A-8C* from distal-to-proximal looking, side, and side-oblique perspectives, respectively. Expansion creates an arrangement of anchors **806** wherein each arm assembly **807, 808** spreads its anchoring ends to define a partial perimeter, for example as described in relation to suturing clip **400.** In the expanded configuration, anchors **806** are held in a position which allows them to be inserted into tissue of the ostium **3** of LAA **1** by manipulation of delivery system **110.**

Closure of suturing clip **800** *(e.g.,* after anchoring) is everting-type, as illustrated in *Figures 8D-8G**.* Eversion of suturing clip **800** is accomplished by further withdrawal of constraint, *e.g.,* by catheter distal end **112,** exposing trunk **811.** Upon removal of constraint, trunk **811** bends *(e.g.,* is superelastically set to bend) through an everting turn (which may be 180° or more).

*Figures 8D-8G* show suturing clip **800** in its collapsed suturing configuration, from side, side-oblique, distal-to-proximal looking, and again side-oblique perspectives, respectively. In *Figures 8D-8E**,* suturing clip **800** is attached to delivery system **110** via delivery mount **113,** while in *Figures 8F-8G**,* it is detached.

As also described for suturing clip **400,** arms **804** of the arm subassemblies **807, 808** are positioned to interdigitate upon collapse to the collapsed suturing configuration. When attached to tissue, complete collapse to the illustrated configuration is potentially resisted by the bulks of two perimeter portions of the ostium **3** encountering each other. As a result, arms **804** continue to exert closing force, potentially helping to ensure a more complete and/or stable closure. The resulting line of closure may be a zigzag band, alternating between positions of the interdigitated arms **804** of the two arm subassemblies **807, 808.**

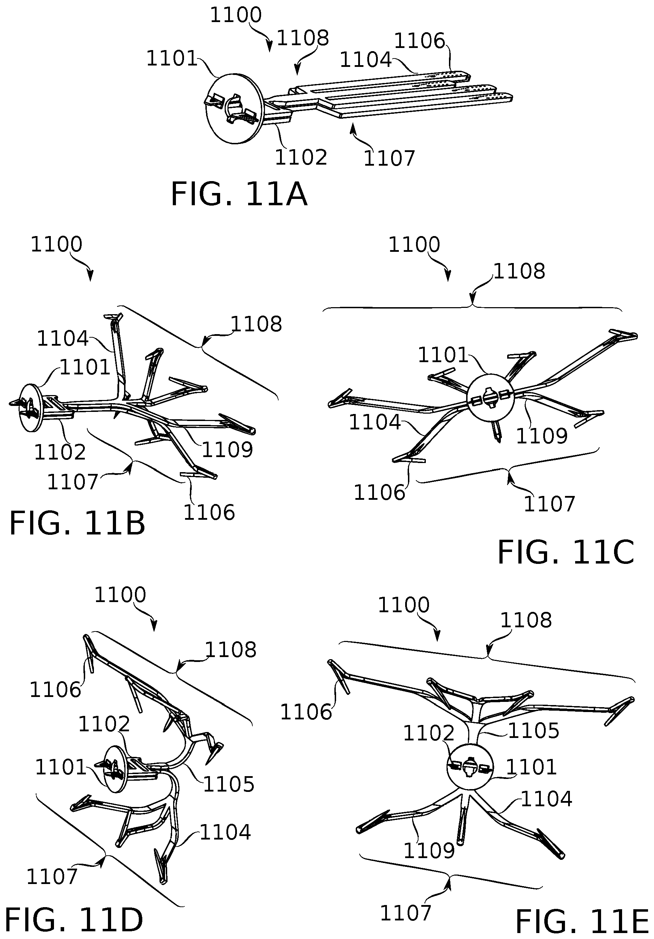

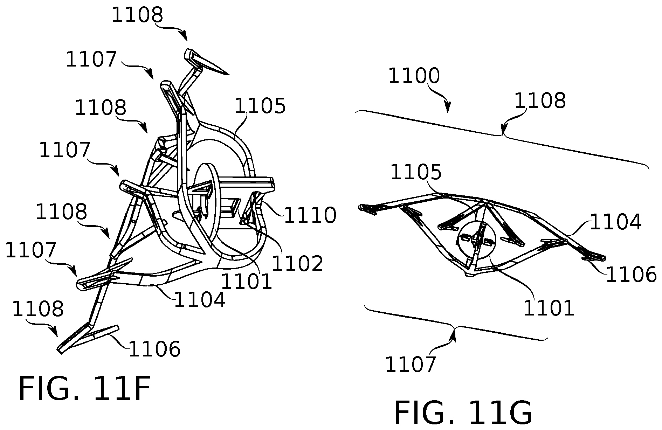

Reference is now made to *Figures 11A-11G**,* which schematically illustrate everting-type closure suturing clip **1100** comprising arm subassemblies **1107, 1108,** according to some embodiments of the present disclosure. Suturing clip **1100** is an embodiment of a suturing clip **100.**

Suturing clip **1100** comprises arm subassemblies **1107, 1108,** each comprising a plurality of arms **1104** joined through an arm assembly trunk **1105** to core **1102.** Conversion from a collapsed delivery configuration (*Figure 11A*) through to a collapsed suturing configuration *(**Figures 11F-11G*) optionally comprises one or both of bending motions of the arms **1104** *(e.g.,* at bends **1109,** and/or along trunk **1105),** and torquing motions of torsion bars **1110.**

In *Figure 11A**,* a collapsed delivery configuration is shown. Arm subassemblies **1107, 1108** are joined at core **1102** by connecting disk **1101.** Optionally, each subassembly **1107, 1108** comprises a separate piece, which may be manufactured by cutting (*e.g*., laser cutting) from sheet of a superelastic material such as nitinol.

Bends of assembly trunk **1105** and along arms **1104** (for example, bend **1109)** are exposed by withdrawal of constraint from catheter distal end **112** of delivery system **110** (not shown in these figures), allowing expansion of suturing clip **1100** to an expanded anchoring configuration.

In some embodiments, an expanded anchoring configuration *(**Figures 11D-11E*) is preceded by a partially-expanded positioning configuration. This configuration is shown in *Figures 11B-11C**.* from side-oblique and distal-to-proximal looking perspectives, respectively.

In the partially-expanded positioning configuration constraint is partially removed, *e.g*., retracted to about the position where each trunk **1105** joins the individual segments of arm subassemblies **1107, 1108.** Arms **1104** expand along a lateral axis to most of their full horizontal extent (*e.g.,* at least 70%, 80%, 90%, or their full horizontal extent. Arms **1104** expand along a vertical axis to a smaller fraction of their eventual full vertical extent, *e.g.,* half or less, for example, about 30%, 40%, or 50% of their fully expanded vertical extent.

This provides a potential advantage by expanding enough to force the suturing clip **1100** to be centered in the LAA, while still remaining sufficiently un-expanded enough that anchoring is not induced. An overtube **111** can be pushed forward again to re-constrain the suturing clip **1100,** and it can be re-collapsed potentially with a low risk of tearing due to being already embedded in tissue (*e.g.,* of the ostium **3** of LAA **1**). This assists repeated expanding a collapsing which may be performed in the course of ensuring that the device is properly positioned before full expansion and device anchoring is established.

A later stage representing an expanded anchoring configuration is shown in *Figures 11D-**11E* from side-oblique and distal-to-proximal looking perspectives, respectively. At a stage of expansion between that of *Figures 11B-11C* and *Figures 11D-11F**,* there may be contact of anchors **1106** with tissue; and then as expansion continues, anchors **1106** "roll over", not only dragging tissue with them, but also potentially gaining further purchase and/or insertion as they change their angle of pulling to one that the tissue resists.

Expansion creates an arrangement of anchors **1106** wherein each arm assembly **1107, 1108** spreads its anchoring ends to define a partial perimeter, for example as described in relation to suturing clip **400.** In the expanded configuration, anchors **1106** are held in a position which allows them to be inserted into tissue of the ostium **3** of LAA **1** by manipulation of delivery system **110.**

*Figures 11F-11G* show suturing clip **1100** in its collapsed suturing configuration, from side-oblique and distal-to-proximal looking perspectives, respectively. Closure of suturing clip **1100** (*e.g.,* after anchoring) is everting-type. Eversion of suturing clip **1100** is accomplished by further withdrawal of constraint, *e.g*., by catheter distal end **112,** exposing trunk **1105.** Upon removal of constraint, trunk **1105** bends (*e.g.,* is superelastically set to bend) through an everting turn (which may be 180° or more).

This motion, in some embodiments, comprise motions induced by torquing of torsion bars **1110.** Comparison of *Figures 11A* and *11F**,* for example, illustrates torsion bars **1110** which have torqued about 90° over the course of deployment. The use of torsion bars **1110** can result in the storage of considerable force by concentrating strain movements into a relatively small area.

As also described for suturing clip **400,** arms **1104** of the arm subassemblies **1107, 1108** are positioned to interdigitate upon collapse to the collapsed suturing configuration. Interdigitation can be with overshoot (to form a pronounced zigzag), or, as shown, toward a more linear configuration. When attached to tissue, complete collapse to the illustrated configuration is potentially resisted by the bulks of two perimeter portions of the ostium **3** encountering each other. As a result, arms **1104** continue to exert closing force, potentially helping to ensure a more complete and/or stable closure. Anchoring along the resulting line of closures alternates between positions of the interdigitated arms **1104** of the two arm subassemblies **1107, 1108.**

### Spreader for Use with a Suturing Clip

Reference is now made to *Figures 9A-9F**,* which illustrate a sequence of operations wherein a tissue spreader **1000** is used together with a delivery system **910** to assist access to LAA **1** for insertion of a suturing clip **100,** according to some embodiments of the present disclosure. Delivery system **910** is an example of a delivery system **110.** Suturing clip **100** is illustrated as having reverting-type closure, similar to suturing clip **300.** It should be understood, however, that features of delivery system **910,** including tissue spreader **1000,** are optionally provided together with any suturing clip design, for example any of the reverting-type closure suturing clips **300, 350, 400,** and/or the everting-type closure suturing clips **500, 800, 1100** described herein.

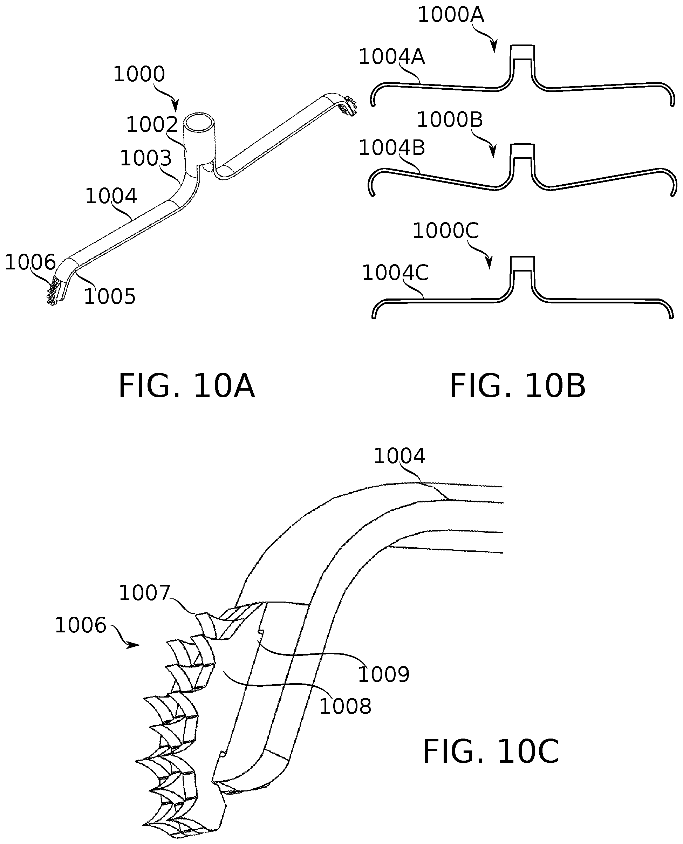

*Figure 9A* illustrates catheter overtube **911.** In *Figure 9B**,* catheter distal end **912** has been advanced distally forward from catheter overtube **911,** with suturing clip **100** inside it. Also advanced distally forward are the arms of spreader **1000,** the parts and operation of which is discussed further in relation to *Figures 10A-10C**.* In *Figure 9C**,* suturing clip **100** is exposed beyond spreader **1000** on delivery mount **913,** after withdrawal of distal catheter end **912.**

In overview, spreader **1000** engages tissue in or near an ostium **3** of an LAA **1** to perform one or more of: (1) opening the aperture (in the direction of spreading) to allow insertion of a suturing clip **100, 2)** shaping the aperture to a shape which is suitable for engagement with the expanded anchoring configuration of the suturing clip **100,** (3) centering and/or orienting the device in the ostium **3** of the LAA **1,** and (4) *closing* the ostium **3** in another direction (which potentially collapses along an axis orthogonal to the direction of spreading), potentially increasing contact force with anchors **106** of suturing clip **100.** The collapse is due to the perimeter of ostium **3** being strained in the direction of spreading.

Once suturing clip **100** is in place (optionally also after anchoring), spreader **1000** is withdrawn again, for example as shown in *Figure 9D**.* Closure of suturing clip **100** continues through stages already described, for example proximal movement of closure disk **120** (*Figure 9E*)*,* and detachment of delivery mount **913** (*Figure 9F*)*.* This closure sequence is optionally substituted in some embodiments, *e*.*g*., by the progressive eversion of a suturing clip **100** having everting-type closure.

Reference is now made to *Figures 10A-10C**,* which schematically illustrate aspects of design of various tissue spreaders **1000, 1000A, 1000B, 1000C,** according to some embodiments of the present disclosure.

*Figure 10A* shows elements of a tissue spreader **1000,** including spreader arms **1004** attached to spreader core **1002.** Each arm **1004** comprises a grip **1006** at its free end. Each arms **1004** further comprises a basal bend **1003** near where arm **1004** attaches to core **1002,** and a distal bend **1005** which re-orients a tip of arm **1004** so that grip **1006** is oriented radially outward to contact lumenal tissue when spreader **1000** is expanded.

Spreader **1000** is formed, in some embodiments, from a superelastic alloy such as nitinol, allowing it to be packaged for delivery within, *e*.*g*., catheter overtube **911.** It is superelastically set to assume the configuration, *e.g.,* of *Figure 10A* when constraint on its expansion is removed.

*Figure 10B* illustrates other arm designs **1004A, 1004B, 1004C** for a tissue spreader **1000A, 1000B, 1000C.** The arm designs differ in aspects of the amount of curvature at their ends (where grip **1006** may be placed), and/or in the angle to which their expanded forms bend relative to a longitudinal axis of a distal portion of delivery system **110.**

With greater end curvature, there is potentially more assurance that a rounded tip surface will encounter tissue, rather than the end, with a concomitant reduction in a potential for accidental perforation. Designs with greater re-curvature beyond perpendicular to the longitudinal axis of the distal portion of delivery system **110** potentially exhibit less stiffness for stretching, but potentially also a reduced chance for perforation.

*Figure 10C* illustrates a design for a grip **1006,** comprising one or more roughened (*e.g.,* ridged and/or toothed) plates **1008.** The plates **1008** are optionally set into an aperture **1009** of arm **1004,** and secured by crimping and/or welding. Optionally, ridges and/or teeth **1007** are offset from each other on adjacent different plates. In some embodiments, roughening of the grip surface is by another method, for example, machining of a thickened end of arm **1004.** Preferably, grip **1006** is rough enough to engage with a surface of the tissue, but not so sharp, penetrating and/or abrasive as to cause it damage.

### General

Reference is now made to *Figure 1A**,* which schematically illustrates a suturing clip **100** engaged to tissue within a LAA **1,** according to some embodiments of the present disclosure.

Reference is also made to *Figure 1B**,* which schematically illustrates the suturing clip **100** of *Figure 1A* in an expanded configuration suitable for tissue engagement, according to some embodiments of the present disclosure.
reference is made to *Figure 1C**,* which schematically illustrates a suturing clip **100** which has engaged tissue within an LAA **1** and then undergone a conformational change to close an LAA ostium **3,** according to some embodiments of the present disclosure.

Reference is also made to *Figure 1D**,* which schematically illustrates the suturing clip **100** of in the LAA-closing conformation of *Figure 1C**,* according to some embodiments of the present disclosure.

Reference is now made to *Figure 1E**,* which schematically illustrates a trans-septal approach insertion of a suturing clip **100** using a delivery system **110,** according to some embodiments of the present disclosure.

Reference is now made to *Figure 1F**,* which is a schematic flowchart of a method of inserting of a suturing clip **100** using a delivery system **110,** according to some embodiments of the present disclosure.

Reference is now made to *Figures 2A-2I**,* which schematically illustrate stages in the deployment of a suturing clip **100,** according to some embodiments of the present disclosure.

Reference is now made to *Figures 2J-2L**,* which schematically illustrate movements **203, 205, 203A, 203B, 205A, 205B** of anchoring positions **201, 201A, 201B** over the course of a transition of a suturing clip **100** between a deployed- and-anchored configuration, and a collapsed suturing configuration, according to some embodiments of the present disclosure.

Reference is now made to *Figures 2M-2P**,* which illustrate details of interactions between a delivery system **110** and a suturing clip **100,** according to some embodiments of the present disclosure.
Reference is now made to *Figure 3A**,* which schematically illustrates an expanded configuration of a reverting core-and-arm assembly **301** of a suturing clip **300,** according to some embodiments of the present disclosure.

Reference is also made to *Figure 3B**,* which schematically illustrates suturing clip **300** an expanded configuration, including closure disk **320,** according to some embodiments of the present disclosure.

Reference is now made to *Figure 3C**,* which schematically illustrates suturing clip **300** in a re-collapsed suturing configuration, collapsed by movement of closure disk **320,** according to some embodiments of the present disclosure, and

Reference is made to *Figure 3D**,* which schematically illustrates suturing closure disk **320,** according to some embodiments of the present disclosure.

Reference is now made to *Figure 3E**,* which schematically illustrates an expanded configuration of a reverting core-and-arm assembly **351** of a suturing clip **350,** according to some embodiments of the present disclosure.

Reference is also made to *Figures 3F-3G**,* which schematically illustrate suturing clip **350** an expanded configuration, including closure disk **370,** according to some embodiments of the present disclosure.
reference is made to *Figure 3H**,* which schematically illustrates suturing clip **350** in a re-collapsed suturing configuration, collapsed by movement of closure disk **370,** according to some embodiments of the present disclosure.

Reference is now made to *Figure 3I*, which schematically illustrates suturing closure disk **370,** according to some embodiments of the present disclosure.

Reference is now made to *Figure 4A**,* which schematically illustrate an expanded configuration of a core-and-arm assembly **401** of a suturing clip **400,** according to some embodiments of the present disclosure.

Reference is also made to *Figure 4B**,* which schematically illustrates suturing clip **400** an expanded configuration, including closure disk **420,** according to some embodiments of the present disclosure.

Reference is made to *Figure 4C**,* which schematically illustrates suturing closure disk **420,** according to some embodiments of the present disclosure.

Reference is now made to *Figures 4D-4H**,* which show deployment of suturing clip **400** under control of a deployment system **410,** including deployment member **415,** according to some embodiments of the present disclosure.

Reference is now made to *Figures 5A-5D**,* which schematically illustrate parts of an everting suturing clip **500,** according to some embodiments of the present disclosure.

Reference is now made to *Figures 5E-5F**,* which schematically illustrate assembled suturing clip **500** in an expanded configuration, according to some embodiments of the present disclosure.

Reference is now made to *Figures 5G-5H**,* which schematically illustrate assembled suturing clip **500** in an everted configuration, according to some embodiments of the present disclosure.

Reference is now made to *Figures 6A-6D**,* which schematically illustrate torsion bar arm mountings, according to some embodiments of the present disclosure.

Reference is now made to *Figures 7A-7F**,* which schematically illustrate suturing clip **500** in three different stages of deployment, each from two different viewing angles, according to some embodiments of the present disclosure.

Reference is now made to *Figure 7G**,* which schematically illustrates a portion of a suturing clip **100** (only one arm is shown) comprising an arm **740** attached to a core **102,** in which bending of arm **740** is around one or more torsion bars **741, 743, 747,** including torsion bars **741, 743** which are positioned along the longitudinal extent of the arm **740.**

Reference is now made to *Figures 8A-8G**,* which schematically illustrate everting-type closure suturing clip **800** comprising arm subassemblies **807, 808,** according to some embodiments of the present disclosure.

Reference is now made to *Figures 9A-9F**,* which illustrate a sequence of operations wherein a tissue spreader **1000** is used together with a delivery system **910** to assist access to LAA **1** for insertion of a suturing clip **100,** according to some embodiments of the present disclosure.

Reference is now made to *Figures 10A-10C**,* which schematically illustrate aspects of design of various tissue spreaders **1000, 1000A, 1000B, 1000C,** according to some embodiments of the present disclosure.

As used herein with reference to quantity or value, the term "about" means "within ±10% of".

The terms "comprises", "comprising", "includes", "including", "having" and their conjugates mean: "including but not limited to".

The term "consisting of" means: "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example" or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the present disclosure may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, embodiments may be presented with reference to a range format. It should be understood that the description in range format is merely for convenience and brevity and should not be construed as an inflexible limitation on the scope of descriptions of the present disclosure. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", *etc.*; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Although descriptions of the present disclosure are provided in conjunction with specific embodiments, it is evident that many alternatives, modifications and variations will be apparent to those skilled in the art. Accordingly, it is intended to embrace all such alternatives, modifications and variations that fall within the broad scope of the disclosure.
In addition, citation or identification of any reference in this application shall not be construed as an admission that such reference is available as prior art to the present disclosure. To the extent that section headings are used, they should not be construed as necessarily limiting.

It is appreciated that certain features which are, for clarity, described in the present disclosure in the context of separate embodiments, may also be provided in combination in a single embodiment. Conversely, various features, which are, for brevity, described in the context of a single embodiment, may also be provided separately or in any suitable subcombination or as suitable in any other described embodiment of the present disclosure. Certain features described in the context of various embodiments are not to be considered essential features of those embodiments, unless the embodiment is inoperative without those elements.

In addition, any priority document(s) of this application is/are hereby cited.

## Claims

1. A suturing clip **(100, 300, 350, 400, 500, 800)** for closing a body lumen aperture comprising:
a plurality of arms **(104)** each joined at a basal side of the arm to a core **(102)** of the suturing clip **(100, 300, 350, 400, 500, 800);** and
a respective anchor **(106)** at a terminal end of each arm **(104);**
wherein the suturing clip **(100, 300, 350, 400, 500, 800)** is configured to convert between:
a collapsed delivery configuration, and
a collapsed suturing configuration shaped to re-position the anchors where they are configured to close tissue of an aperture perimeter after said tissue is attached by contact with the anchors in an expanded anchoring configuration;
wherein the suturing clip **(100, 300, 350, 400, 500, 800),** in the collapsed delivery configuration, is configured to attach to a delivery mount and configured to be advanced on the delivery mount **(113)** by distal movement along a proximal-to-distal axis of the suturing clip **(100, 300, 350, 400, 500, 800);**
**characterized in that**
said arms **(104)** of the suturing clip **(100, 300, 350, 400, 500, 800),** in said collapsed delivery configuration, are configured to be extended in a proximal-distal configuration in relation to said core **(102)** along a proximal-to-distal axis;
wherein said arms **(104)** of the suturing clip **(100, 300, 350, 400, 500, 800)** are configured to transition from a proximal-distal configuration to a distal-proximal configuration in relation to said core **(102)** during a transition between said collapsed delivery configuration to said collapsed suturing configuration; and
wherein the anchors **(106)** of the suturing clip **(100, 300, 350, 400, 500, 800),** in the collapsed suturing configuration, are configured to be positioned proximal to the core **(102)** along the proximal-to-distal axis.

2. The suturing clip **(100, 300, 350, 400, 500, 800)** of claim 1, wherein the suturing clip **(100, 300, 350, 400, 500, 800)** is configured to resiliently self-expand from the collapsed delivery configuration to the expanded anchoring configuration having a maximum diameter at least 5x larger than a maximum diameter of the suturing clip **(100, 300, 350, 400, 500, 800)** in the collapsed delivery configuration.

3. The suturing clip **(100, 300, 350, 400, 500, 800)** of claim 1, together with the delivery mount **(113)** attached to the suturing clip **(100, 300, 350, 400, 500, 800),** and wherein the delivery mount **(113)** is configured to be detached from the suturing clip **(100, 300, 350, 400, 500, 800)** in the collapsed suturing configuration and withdrawn through tissue closed by the anchors **(106)** of the suturing clip **(100, 300, 350, 400, 500, 800).**

4. The suturing clip **(100, 300, 350, 400, 500, 800)** of any one of claims 1-3, sized to be sheathed by a sheathing lumen of a trans-vascular catheter; and provided together with the sheathing lumen and trans-vascular catheter, wherein the suturing clip **(100, 300, 350, 400, 500, 800)** in the collapsed delivery configuration is sheathed within the sheathing lumen.

5. The suturing clip **(100, 300, 350, 400, 500, 800)** of claim 4, wherein:
the suturing clip **(100, 300, 350, 400, 500, 800)** is configured to resiliently self-expand from the collapsed delivery configuration to the expanded anchoring configuration upon a partial unsheathing from the sheathing lumen; and
the suturing clip **(100, 300, 350, 400, 500, 800)** is configured to resiliently self-collapse from the expanded anchoring configuration to the collapsed suturing configuration upon a further unsheathing from the sheathing lumen.

6. The suturing clip **(100, 300, 350, 400, 500, 800)** of claim 5, wherein the suturing clip **(100, 300, 350, 400, 500, 800)** is configured to evert during converting between the collapsed delivery configuration and the expanded anchoring configuration, so that a side of the anchors **(106)** which face radially inward in the collapsed delivery configuration faces outward in the expanded anchoring configuration.

7. The suturing clip **(100, 300, 350, 400, 500, 800)** of any one of claim 5-6, having a proximal-to-distal axis, wherein the suturing clip **(100, 300, 350, 400, 500, 800)** is configured to evert during converting between the collapsed delivery configuration and the collapsed suturing configuration, so that a portion of the arms **(104)** which is initially on a distal side of the core **(102)** moves to a proximal side of the core **(102).**

8. The suturing clip **(100, 300, 350, 400, 500, 800)** of claim 5, wherein conversion from the expanded anchoring configuration to the collapsed suturing configuration comprises a reverting movement of the arms **(104)** in a direction opposite a direction of their expansion from the collapsed delivery configuration to the expanded anchoring configuration.

9. The suturing clip **(100, 300, 350, 400, 500, 800)** of any one of claims 4-7, together with a spreader **(1000),** separately actuatable to extend laterally from the trans-vascular catheter and configured to contact tissue of the body lumen aperture with grips **(1006)** to set a position of the sheathing lumen from which the suturing clip **(100, 300, 350, 400, 500, 800)** is deployed.

10. The suturing clip **(100, 300, 350, 400, 500, 800)** of any one of claims 1-9, wherein the anchors **(106),** in the collapsed suturing configuration, define a zigzag pattern extending along a band of closure of the closed tissue.

11. The suturing clip **(100, 300, 350, 400, 500, 800)** of any one of claims 1-10, wherein a first plurality of the arms extends from the core **(102)** via a trunk which is shared in common by the plurality of the arms.

12. The suturing clip **(100, 300, 350, 400, 500, 800)** of claim 11, comprising a second plurality of arms, and wherein arms of the first plurality are configured to interdigitate with arms of the second plurality in the collapsed suturing configuration.

13. The suturing clip **10(100, 300, 350, 400, 500, 800) 0** of any one of claims 1-12, comprising a torsion bar **741, 743, 747,** wherein at least one of the arms **(104)** is mounted to at least one side of the torsion bar, and the torsion bar is configured to twist to move the at least one of the arms **(104)** from the collapsed delivery configuration toward the collapsed suturing configuration.

14. The suturing clip **(100, 300, 350, 400, 500, 800)** of claim 1, wherein the anchors **(106)** are positioned on a radially inward side of the arms **(104)** in the collapsed delivery configuration, and move to a radially outward side of the arms **(104)** during movement of the arms **(104)** toward the collapsed suturing configuration.

## Patentansprüche

1. Eine Nahtklammer (**100, 300, 350, 400, 500, 800**) zum Verschließen einer Körperlumenöffnung, umfassend:
eine Vielzahl von Armen (**104**), die jeweils an einer Basalseite des Arms mit einem Kern (**102**) der Nahtklammer **(100, 300, 350, 400, 500, 800)** verbunden sind; und
einen jeweiligen Anker (**106**) an einem Endpunkt jedes Arms **(104)**;
wobei die Nahtklammer **(100, 300, 350, 400, 500, 800)** so konfiguriert ist, dass sie zwischen folgenden Konfigurationen umschaltet:
einer zusammengeklappten Zuführungskonfiguration und
einer zusammengeklappten Nahtkonfiguration, die so ausgeformt ist, dass sie die Anker umpositioniert, wo sie so konfiguriert sind, dass sie ein Gewebe eines Öffnungsumfangs schließen, nachdem das Gewebe durch Kontakt mit den Ankern in einer expandierten Ankerkonfiguration befestigt ist;
wobei die Nahtklammer **(100, 300, 350, 400, 500, 800)** in der zusammengeklappten Zuführungskonfiguration so konfiguriert ist, dass sie an einer Zuführungshalterung befestigt werden kann und auf der Zuführungshalterung (**113**) durch eine distale Bewegung entlang einer von proximal nach distal verlaufenden Achse der Nahtklammer **(100, 300, 350, 400, 500, 800)** vorgeschoben werden kann;
**dadurch gekennzeichnet, dass**
die Arme **(104)** der Nahtklammer **(100, 300, 350, 400, 500, 800)** in der zusammengeklappten Zuführungskonfiguration so konfiguriert sind, dass sie in einer proximal-distalen Konfiguration in Bezug auf den Kern **(102)** entlang einer von proximal nach distal verlaufenden Achse ausgedehnt werden können;
wobei die Arme **(104)** der Nahtklammer **(100, 300, 350, 400, 500, 800)** so konfiguriert sind, dass sie während eines Übergangs von der zusammengeklappten Zuführungskonfiguration zur zusammengeklappten Nahtkonfiguration in Bezug auf den Kern **(102)** von einer proximal-distalen Konfiguration in eine distal-proximale Konfiguration übergehen; und
wobei die Anker **(106)** der Nahtklammer **(100, 300, 350, 400, 500, 800)** in der zusammengeklappten Nahtkonfiguration so konfiguriert sind, dass sie entlang der von proximal nach distal verlaufenden Achse proximal zum Kern **(102)** positioniert sind.

2. Nahtklammer **(100, 300, 350, 400, 500, 800)** nach Anspruch 1, wobei die Nahtklammer **(100, 300, 350, 400, 500, 800)** so konfiguriert ist, dass sie sich elastisch aus der zusammengeklappten Zuführungskonfiguration in die expandierte Ankerkonfiguration ausfährt, die einen maximaler Durchmesser aufweist, der mindestens fünfmal größer ist als ein maximaler Durchmesser der Nahtklammer **(100, 300, 350, 400, 500, 800)** in der zusammengeklappten Zuführungskonfiguration.

3. Nahtklammer **(100, 300, 350, 400, 500, 800)** nach Anspruch 1, zusammen mit der Zuführungshalterung **(113)**, die an der Nahtklammer **(100, 300, 350, 400, 500, 800)** befestigt ist, und wobei die Zuführungshalterung **(113)** so konfiguriert ist, dass sie von der Nahtklammer **(100, 300, 350, 400, 500, 800)** in der zusammengeklappten Nahtkonfiguration abgenommen und durch das von den Ankern (**106**) der Nahtklammer **(100, 300, 350, 400, 500, 800)** verschlossene Gewebe zurückgezogen werden kann.

4. Nahtklammer **(100, 300, 350, 400, 500, 800)** nach einem der Ansprüche 1-3, die so dimensioniert ist, dass sie von einem Mantel-Lumen eines transvaskulären Katheters ummantelt werden kann; und die zusammen mit dem Mantel-Lumen und dem transvaskulären Katheter bereitgestellt wird, wobei die Nahtklammer **(100, 300, 350, 400, 500, 800)** in der zusammengeklappten Zuführungskonfiguration innerhalb des Mantel-Lumens umhüllt ist.

5. Nahtklammer **(100, 300, 350, 400, 500, 800)** nach Anspruch 4, wobei:
die Nahtklammer **(100, 300, 350, 400, 500, 800)** so konfiguriert ist, dass sie sich bei einem teilweisen Herausziehen aus dem Mantel-Lumen elastisch von der zusammengeklappten Zuführungskonfiguration in die expandierte Ankerkonfiguration selbst expandiert; und
die Nahtklammer **(100, 300, 350, 400, 500, 800)** so konfiguriert ist, dass sie sich bei einem weiteren Herausziehen aus dem Mantel-Lumen elastisch von der expandierten Ankerkonfiguration in die zusammengeklappte Nahtkonfiguration zusammenklappt.

6. Nahtklammer **(100, 300, 350, 400, 500, 800)** nach Anspruch 5, wobei die Nahtklammer **(100, 300, 350, 400, 500, 800)** so konfiguriert ist, dass sie sich beim Übergang zwischen der zusammengeklappten Zuführungskonfiguration und der expandierten Ankerkonfiguration umstülpt, sodass eine Seite der Anker **(106)**, die in der zusammengeklappten Zuführungskonfiguration radial nach innen gerichtet ist, in der expandierten Ankerkonfiguration nach außen gerichtet ist.

7. Nahtklammer **(100, 300, 350, 400, 500, 800)** nach einem der Ansprüche 5-6, die eine von proximal nach distal verlaufende Achse aufweist, wobei die Nahtklammer **(100, 300, 350, 400, 500, 800)** so konfiguriert ist, dass sie sich beim Übergang von der zusammengeklappten Zuführungskonfiguration in die zusammengeklappte Nahtkonfiguration umstülpt, sodass sich ein Bereich der Arme **(104)**, der sich anfänglich auf einer distalen Seite des Kerns (**102**) befindet, auf eine proximale Seite des Kerns **(102)** bewegt.

8. Nahtklammer **(100, 300, 350, 400, 500, 800)** nach Anspruch 5, wobei ein Übergang von der expandierten Ankerkonfiguration in die zusammengeklappte Nahtkonfiguration eine Rückstellbewegung der Arme **(104)** in einer Richtung umfasst, die der Richtung ihrer Expansion von der zusammengeklappten Zuführungskonfiguration in die expandierte Ankerkonfiguration entgegengesetzt ist.

9. Nahtklammer **(100, 300, 350, 400, 500, 800)** nach einem der Ansprüche 4-7, zusammen mit einem Spreizer (**1000**), der separat betätigbar ist, um sich seitlich aus dem transvaskulären Katheter zu erstrecken, und der so konfiguriert ist, dass er mit Greifern (**1006**) das Gewebe der Körperlumenöffnung berührt, um eine Position des Mantel-Lumens festzulegen, aus der die Nahtklammer **(100, 300, 350, 400, 500, 800)** eingesetzt wird.

10. Nahtklammer **(100, 300, 350, 400, 500, 800)** nach einem der Ansprüche 1-9, wobei die Anker **(106)** in der zusammengeklappten Nahtkonfiguration ein Zickzackmuster definieren, das sich entlang eines Verschlussstreifens des geschlossenen Gewebes erstreckt.

11. Nahtklammer **(100, 300, 350, 400, 500, 800)** nach einem der Ansprüche 1 bis 10, wobei sich eine erste Vielzahl der Arme vom Kern (**102**) über einen Stamm erstreckt, der von der Vielzahl der Arme gemeinsam genutzt wird.

12. Nahtklammer **(100, 300, 350, 400, 500, 800)** nach Anspruch 11, umfassend eine zweite Vielzahl von Armen, wobei die Arme der ersten Vielzahl so konfiguriert sind, dass sie in der zusammengeklappten Nahtkonfiguration mit den Armen der zweiten Vielzahl ineinandergreifen.

13. Nahtklammer **10(100, 300, 350, 400, 500, 800) 0** nach einem der Ansprüche 1-12, umfassend eine Torsionsstange **741**, **743**, **747**, wobei mindestens einer der Arme **(104)** an mindestens einer Seite der Torsionsstange montiert ist und die Torsionsstange so konfiguriert ist, dass sie sich verdreht, um den mindestens einen der Arme **(104)** aus der zusammengeklappten Zuführungskonfiguration in Richtung der zusammengeklappten Nahtkonfiguration zu bewegen.

14. Nahtklammer **(100, 300, 350, 400, 500, 800)** nach Anspruch 1, wobei die Anker **(106)** in der zusammengeklappten Zuführungskonfiguration auf einer radial inneren Seite der Arme **(104)** positioniert sind und sich während der Bewegung der Arme **(104)** in Richtung der zusammengeklappten Nahtkonfiguration auf eine radial äußere Seite der Arme **(104)** bewegen.

## Revendications

1. Agrafe de suture (**100, 300, 350, 400, 500, 800**) destinée à fermer une ouverture de lumière corporelle, comprenant :
une pluralité de bras (**104**) reliés chacun, à un niveau de leur face basale, à un noyau (**102**) de l'agrafe de suture **(100, 300, 350, 400, 500, 800)** ; et
un ancrage respectif (**106**) à un extrémité terminale de chaque bras **(104)** ;
dans laquelle l'agrafe de suture **(100, 300, 350, 400, 500, 800)** est configurée pour la conversion entre:
une configuration de mise en place repliée, et
une configuration de suture repliée conçue pour repositionner les ancrages de manière à ce qu'ils soient configurés pour refermer des tissus formant un périmètre d'ouverture, une fois que ledit tissu a été fixé par contact avec les ancrages dans une configuration d'ancrage déployée ;
dans laquelle l'agrafe de suture **(100, 300, 350, 400, 500, 800)**, dans la configuration de mise en place repliée, est conçue pour se fixer à un support de mise en place et pour être avancée sur ce support de mise en place (**113**) par un mouvement distal le long d'un axe proximal-distal de l'agrafe de suture **(100, 300, 350, 400, 500, 800)** ;
**caractérisée en ce que**
lesdits bras **(104)** de l'agrafe de suture **(100, 300, 350, 400, 500, 800)**, dans ladite configuration de mise en place repliée, sont conçus pour être déployés selon une configuration proximale-distale par rapport audit noyau **(102)** le long d'un axe proximal-distal ;
dans laquelle lesdits bras **(104)** de l'agrafe de suture **(100, 300, 350, 400, 500, 800)** sont configurés pour passer d'une configuration proximale-distale à une configuration distale-proximale par rapport audit noyau **(102)** lors d'une transition entre ladite configuration de mise en place repliée et ladite configuration de suture repliée ; et
dans laquelle les ancrages **(106)** de l'agrafe de suture **(100, 300, 350, 400, 500, 800)**, dans la configuration de suture repliée, sont configurés pour être positionnés à proximité du noyau **(102)** le long de l'axe proximal-distal.

2. L'agrafe de suture **(100, 300, 350, 400, 500, 800)** selon la revendication 1, dans laquelle l'agrafe de suture **(100, 300, 350, 400, 500, 800)** est conçue pour s'auto-déployer de manière élastique depuis la configuration de mise en place repliée vers la configuration d'ancrage déployée présentant un diamètre maximal au moins 5 fois supérieur à un diamètre maximal de l'agrafe de suture **(100, 300, 350, 400, 500, 800)** dans la configuration de mise en place repliée.

3. L'agrafe de suture **(100, 300, 350, 400, 500, 800)** selon la revendication 1, associée au support de mise en place (**113**) fixé à l'agrafe de suture **(100, 300, 350, 400, 500, 800)**, et dans laquelle le support de mise en place **(113)** est configuré pour être détaché de l'agrafe de suture **(100, 300, 350, 400, 500, 800)** dans la configuration de suture repliée et retiré à travers du tissu fermé par les ancrages (**106**) de l'agrafe de suture **(100, 300, 350, 400, 500, 800)**.

4. L'agrafe de suture **(100, 300, 350, 400, 500, 800)** selon l'une quelconque des revendications 1 à 3, dimensionnée pour être logée dans une lumière de gaine d'un cathéter transvasculaire ; et fournie conjointement avec la lumière de gaine et le cathéter transvasculaire, dans laquelle l'agrafe de suture **(100, 300, 350, 400, 500, 800)** dans la configuration de mise en place repliée est logée à l'intérieur de la lumière de gaine.

5. L'agrafe de suture **(100, 300, 350, 400, 500, 800)** selon la revendication 4, dans laquelle :
l'agrafe de suture **(100, 300, 350, 400, 500, 800)** est configurée pour s'expanser de manière élastique et autonome, passant de la configuration de mise en place repliée à la configuration d'ancrage déployée, lors d'un désenrobage partiel de la lumière de la gaine ; et
l'agrafe de suture **(100, 300, 350, 400, 500, 800)** est configurée pour se replier de manière élastique, passant de la configuration d'ancrage déployée à la configuration de suture repliée, lors d'un désenrobage supplémentaire hors de la lumière de la gaine.

6. L'agrafe de suture **(100, 300, 350, 400, 500, 800)** selon la revendication 5, dans laquelle l'agrafe de suture **(100, 300, 350, 400, 500, 800)** est configurée pour s'éverser lors de la conversion entre la configuration de mise en place repliée à la configuration d'ancrage déployée, de sorte qu'un côté des ancrages **(106)** qui est orienté radialement vers l'intérieur dans la configuration de mise en place repliée soit orienté vers l'extérieur dans la configuration d'ancrage déployée.

7. L'agrafe de suture **(100, 300, 350, 400, 500, 800)** selon l'une quelconque des revendications 5 à 6, présentant un axe proximal-distal, dans laquelle l'agrafe de suture **(100, 300, 350, 400, 500, 800)** est configurée pour s'éverser lors de la conversion entre la configuration de mise en place repliée et la configuration de suture déployée, de sorte qu'une partie des bras **(104)** qui se trouve initialement d'un côté distal du noyau (**102**) se déplace vers un côté proximal du noyau **(102)**.

8. L'agrafe de suture **(100, 300, 350, 400, 500, 800)** selon la revendication 5, dans laquelle de la conversion de la configuration d'ancrage déployée à la configuration de suture repliée comprend un mouvement de retour des bras **(104)** dans une direction opposée à une direction de leur déploiement de la configuration de mise en place repliée à la configuration d'ancrage déployée.

9. L'agrafe de suture **(100, 300, 350, 400, 500, 800)** selon l'une quelconque des revendications 4 à 7, associée à un écarteur (**1000**), actionnable séparément pour s'étendre latéralement à partir du cathéter transvasculaire et configuré pour entrer en contact avec du tissu de l'ouverture de lumière corporelle à l'aide de pinces (**1006**) afin de définir une position de la lumière de gainage à partir de laquelle l'agrafe de suture **(100, 300, 350, 400, 500, 800)** est déployée.

10. L'agrafe de suture **(100, 300, 350, 400, 500, 800)** selon l'une quelconque des revendications 1 à 9, dans laquelle les ancrages **(106)**, dans la configuration de suture repliée, définissent un motif en zigzag s'étendant le long d'une bande de fermeture du tissu fermé.

11. L'agrafe de suture **(100, 300, 350, 400, 500, 800)** selon l'une quelconque des revendications 1 à 10, dans laquelle une première pluralité des bras s'étend à partir du noyau (**102**) via un tronc commun à cette pluralité de bras.

12. L'agrafe de suture **(100, 300, 350, 400, 500, 800)** selon la revendication 11, comprenant une deuxième pluralité de bras, et dans laquelle des bras de la première pluralité sont configurés pour s'imbriquer avec des bras de la deuxième pluralité dans la configuration de suture repliée.

13. L'agrafe de suture **(100, 300, 350, 400, 500, 800)** selon l'une quelconque des revendications 1 à 12, comprenant une barre de torsion **(741**, **743**, **747)**, dans laquelle au moins l'un des bras **(104)** est monté sur au moins un côté de la barre de torsion, et la barre de torsion est configurée pour se tordre afin de déplacer ledit au moins un bras **(104)** de la configuration de mise en place repliée vers la configuration de suture repliée.

14. L'agrafe de suture **(100, 300, 350, 400, 500, 800)** selon la revendication 1, dans laquelle les ancrages **(106)** sont positionnés sur un côté radialement intérieur des bras **(104)** dans la configuration de mise en place repliée, et se déplacent vers un côté radialement extérieur des bras **(104)** lors du déplacement des bras **(104)** vers la configuration de suture repliée.
